(19) Europäisches Patentamt
European Patent Office
Office européen des brevets

(11) EP 1 746 165 A1

(12) EUROPEAN PATENT APPLICATION

(43) Date of publication:
**24.01.2007 Bulletin 2007/04**

(51) Int Cl.:
***C12P 21/02*** *(2006.01)* ***C07K 14/08*** *(2006.01)*
***C12N 15/72*** *(2006.01)*

(21) Application number: **05106729.6**

(22) Date of filing: **21.07.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Cytos Biotechnology AG**
**8952 Schlieren (CH)**

(72) Inventors:
- **Hennecke, Frank**
  **8305 Dietlikon (CH)**
- **Rhiel, Martin**
  **8906 Bonstetten (CH)**
- **Emmerling, Marcel**
  **8952 Schlieren (CH)**
- **Steiner, Philipp**
  **8952 Schlieren (CH)**

(74) Representative: **Sperrle, Martin et al**
**Cytos Biotechnology AG,**
**Wagistrasse 25**
**8952 Schlieren (CH)**

# (54) Scalable fermentation process

(57) This invention provides a robust fermentation process for the expression of a capsid protein of a bacteriophage which is forming a VLP by self-assembly, wherein the process is scalable to a commercial production scale and wherein the expression rate of the capsid protein is limited to obtain improved yield of soluble capsid protein. This is achieved by combining the advantages of fed-batch culture and of lactose induced expression systems with specific process parameters providing improved repression of the promoter during the growth phase and high plasmid retention throughout the process.

EP 1 746 165 A1

## Description

FIELD OF THE INVENTION

This invention is related to the field of protein expression and fermentation technology. A process for the efficient expression of recombinant bacteriophage capsid protein in a bacterial host is described. The process leads to high yield of recombinant capsid protein which is capable of forming a virus-like particle (VLP) by self-assembly. Furthermore, the process is scalable from laboratory scale to fermenter volumes larger than 50 litres.

BACKGROUND OF THE INVENTION

Recent vaccination strategies exploit the immunogenicity of viruses or virus-like-particles (VLPs) to enhance the immune response towards antigens. For example, WO02/056905 demonstrates the utility of VLPs as a carrier to present antigens linked thereto in a highly ordered repetitive array. As a component of a vaccine composition, such antigen arrays can cause a strong immune response, in particular antibody responses, against the antigen which is strong enough to overcome even the immune system's inherent tolerance towards self antigens. Such antigen arrays are therefore useful in the production of vaccines for the treatment of infectious diseases and allergies as well as for the efficient induction of self-specific immune responses, e.g. for the treatment of cancer, rheumatoid arthritis and various other diseases.

Capsid proteins of bacteriophages are particularly suited as antigen carrier and have been shown to efficiently self-assemble into VLPs upon expression in a bacterial host (Kastelein et al. 1983, Gene 23:245-254; Kozlovskaya et al. 1986, Dokl. Akad. Nauk SSSR 287:452-455). Moreover, capsid proteins of bacteriophages such as derived from fr (Pushko et al. 1993, Protein Engineering 6(8)883-891), Qβ (Kozlovska et al. 1993, Gene 137:133-137; Ciliens et al. 2000, FEBS Letters 24171:1-4; Vasiljeva et al 1998, FEBS Letters 431:7-11) and MS-2 (WO92/13081; Mastico et al. 1993, Journal of General Virology 74:541-548; Heal et al. 2000, Vaccine 18:251-258) have been produced in bacterial hosts using inducible promoters such as the trp promoter or a trp-T7 fusion (in the case of fr and Qb) or the tac promoter using IPTG as inducer substance (in the case of MS-2). The use of inducible promoters is beneficial, to avoid possible toxic effects of the recombinant capsid protein and the metabolic burden of protein expression which both might reduce the growth of the bacterial expression host and, ultimately, the yield of expressed protein.

However, the expression systems used so far for the expression of capsid proteins of bacteriophages have been applied in small scale fermentations, i.e. in laboratory scale and small batch cultures with volumes of typically clearly below 1 liter. An upscale of these systems comprising volumes of 50 liter and more is expected to diminish in a great extent the respective capsid protein yield due to increased promoter leakage and/or lowered plasmid retention.

A further problem associated with commercially desired high-level expression and rapid accumulation of recombinant capsid proteins of bacteriophages is the formation of incorrectly folded protein species and the formation of so called inclusion bodies, i.e. protein aggregates, which are insoluble and which may hamper further downstream processes. Thus, for bacteriophage MS-2 coat protein the formation of protein aggregates and of protein species which lost their ability to self-assemble to VLPs have been reported when the protein was expressed under the control of the strong T7 promoter after IPTG induction using the pET expression system (Peabody & Al-Bitar 2001, Nucleic Acid Research 29(22):e113).

High expression rates of the recombinant capsid protein may therefore have a negative impact on the yield of correctly assembled VLPs. The production of VLP-based vaccines in a commercial scale requires, therefore, the establishment of an efficient, and in particular scalable fermentation process for the expression of recombinant capsid protein of bacteriophages leading to a product of constant quality and purity having the capability of self-assembling into VLPs, whereby the formation of insoluble fractions of the capsid protein is minimised or avoided.

Therefore, it is an object of the present invention to provide a process for expression of a recombinant capsid protein of a bacteriophage which avoids or minimizes the disadvantage or disadvantages of the prior art processes, and in particular, which is scalable to a commercial scale and still leading to a product of constant quality and purity and the capability of self-assemblance to VLPs, and wherein the formation of insoluble fraction of the capsid protein is minimised or avoided.

SUMMARY OF THE INVENTION

The invention relates to a process for expression of a recombinant capsid protein of a bacteriophage, or a mutant or fragment thereof being capable of forming a VLP by self assembly, said process comprising the steps of: a.) introducing an expression plasmid into a bacterial host, wherein said expression plasmid comprises a nucleotide sequence encoding said recombinant capsid protein, or mutant or fragment thereof, and a promoter being inducible by lactose, and wherein said bacterial host overexpresses the repressor lacI; b.) introducing said bacterial host into a batch culture; c.) cultivating said bacterial host in a medium comprising a major carbon source and under conditions under which said promoter is repressed by lacI; feeding said batch culture with said major carbon source; and e.) inducing said promoter with an inducer, wherein preferably said feeding of said batch culture with said major carbon source is continued.

This invention provides a robust fermentation process for the expression of a capsid protein of a bacteriophage which is forming a VLP by self-assembly, wherein the process is scalable to a commercial production scale and wherein the expression rate of the capsid protein leads to improved yield of soluble capsid protein. This is, in particular, achieved by improved repression of the promoter during the growth phase and high plasmid retention throughout the process. The expression system further avoids formation of insoluble protein aggregates by limiting the maximum expression rate occurring during the production phase.

In a preferred embodiment said bacteriophage is a RNA phage. More preferably, said RNA phage is selected from the group consisting of: a.) bacteriophage Qβ; b.) bacteriophage AP205; c.) bacteriophage fr; d.) bacteriophage GA; e.) bacteriophage SP; f.) bacteriophage MS2; g.) bacteriophage M11; h.) bacteriophage MX1; i.) bacteriophage NL95; j.) bacteriophage f2; k.) bacteriophage PP7 and 1.) bacteriophage R17. Preferably, said RNA phage is Qβ. More preferably said recombinant capsid protein comprises or alternatively consists of an amino acid sequence selected from the group consisting of SEQ ID NO:5, SEQ ID NO:7, SEQ ID NO:8, SEQ ID NO:9, SEQ ID NO: 10, and SEQ ID NO: 11. Still more preferably said recombinant capsid protein comprises SEQ ID NO:5, most preferably said recombinant capsid protein consists of SEQ ID NO:5.

In a further preferred embodiment said recombinant capsid protein comprises or alternatively consists of an amino acid sequence selected from the group consisting of SEQ ID NO:12, SEQ ID NO:13, and SEQ ID NO:14. More preferably said recombinant capsid protein comprises SEQ ID NO:12, most preferably said recombinant capsid protein consists of SEQ ID NO:12.

In another embodiment of the present invention, said expression plasmid comprises a first stop codon, and wherein said first stop codon is TAA, and wherein preferably said TAA is located directly 3' of said nucleotide sequence.

In a further embodiment said expression plasmid comprises a first stop codon and a second stop codon, wherein said first stop codon is located directly 3' of said nucleotide sequence and wherein said second stop codon is located directly 3' of said first stop codon, and wherein at least one of said first or second stop codon is TAA.

In a further embodiment said expression plasmid comprising said nucleotide sequence comprises an additional nucleotide sequence, wherein said nucleotide sequence is encoding Qβ CP, or a mutant or fragment thereof, and wherein said additional nucleotide sequence is encoding the Qβ A1 protein or a mutant or fragment thereof, and wherein said nucleotide sequence and said additional nucleotide sequence are separated by exactly one sequence stretch comprising at least one TAA stop codon. In a preferred embodiment said expression plasmid comprises the nucleotide sequence of SEQ ID NO:6 or, more preferably, has the nucleotide sequence of SEQ ID NO: 1.

In one embodiment of the invention the said promoter is selected from the group consisting of the a.) tac promoter; b.) trc promoter; c.) tic promoter; d.) lac promoter; e.) lacUV5 promoter; f.) $P_{syn}$ promoter; g.) $lpp^a$ promoter; h.) lpp-lac romoter; i.) T7-lac promoter; j.) T3-lac promoter; k.) T5-lac promoter; and l.) a promoter having at least 50 % sequence homology to SEQ ID NO:2. In a preferred embodiment said promoter has at least 50 %, 60 %, 70 %, 80, 90, or 95%, preferably 98 to 100 %, most preferably 99 % sequence homology to SEQ ID NO:2. In a further preferred embodiment said promoter is selected from the group consisting of tic promoter, trc promoter and tac promoter. Even more preferably said promoter is the tac promoter. Most preferably said promoter comprises or alternatively consists of the nucleotide sequence of SEQ ID NO:2.

In one embodiment said major carbon source is glucose or glycerol, preferably glycerol.

In one embodiment said feeding of said batch culture is performed with a flow rate, wherein said flow rate increases with an exponential coefficient $\mu$, and wherein preferably said exponential coefficient $\mu$ is below $\mu_{max}$.

In a further embodiment said inducing of said promoter in step e.) is performed by co-feeding said batch culture with said inducer, preferably lactose and said major carbon source, preferably glycerol, at a constant flow rate.

In a further embodiment said inducing of said promoter in step e.) of claim 1 is performed by co-feeding said batch culture with said inducer, preferably lactose and said major carbon source, preferably glycerol, at an increasing flow rate.

In a further embodiment said inducer is lactose, wherein preferably said lactose and said major carbon source are co-fed to said batch culture in a ratio of about 2:1 to 1:3 (w/w).

In a further embodiment said inducer is IPTG wherein preferably the concentration of said IPTG in said batch culture is 0.001 to 5 mM, preferably 0.001 to 1 mM, more preferably 0.005 to 1 mM, still more preferably 0.005 to 0.5 mM. In a very preferred embodiment the concentration of IPTG is about 0.01 mM, most preferrably 0.01 mM.

In one embodiment said bacterial host comprises the $lacI^q$ gene or the lacQ1 gene, preferably the $lacI^q$ gene. In a preferred embodiment the $lacI^q$ gene or the lacQ1 gene are located on the chromosome of the bacterial host. In a further prefered embodiment the $lacI^q$ gene or the lacQ1 gene are located on a plasmid, preferably on the plasmid carrying the expression construct.

In one embodiment said bacterial host is selected from the group consisting of the strains E. coli RB791, E. coli DH20 and E. coli Y1088. Preferably said bacterial host is E. coli RB791.

In one embodiment said bacterial host comprises β-galactosidase activity.

In one embodiment said cultivating and feeding of said batch culture and said inducing of said promoter is performed at a temperature which is below the optimal growth temperature of said bacterial host. Preferably said temperature is between 23 °C and 35 °C, more preferably between 25 and 33 °C, even more preferably between 27 and 32 °C, still more preferably between 28 and 31 °C. Even more preferably said temperature is about 30 °C, most preferably said temperature is 30 °C.

In one embodiment said cultivating and feeding of said batch culture is performed at a temperature which is below the optimal growth temperature of said bacterial host, wherein preferably said temperature is between 23 °C and 35 °C, more preferably between 25 and 33 °C, even more preferably between 27 and 32 °C, still more preferably between 28 and 31 °C, even more preferably said temperature is about 30 °C, most preferably said temperature is 30 °C, and said inducing of said promoter is performed at the optimal growth temperature of the bacterial host, preferably at about 37°C.

In one embodiment said cultivating and feeding of said batch culture and said inducing of said promoter is performed in the absence of an antibiotic.

In a specific embodiment said expression plasmid has the nucleotide sequence of SEQ ID NO: 1, said major carbon source is glycerol, said feeding of said batch culture is performed with a flow rate, wherein said flow rate increases with an exponential coefficient $\mu$, and wherein said exponential coefficient $\mu$ is below $\mu_{max}$, said inducing of said promoter by co-feeding said batch culture is performed with a constant flow rate, wherein lactose and glycerol are co-fed to the batch culture in a ratio of about 2:1 to 1:3 (w/w), preferably about 1:1 (w/w), and wherein said cultivating and feeding of said batch culture and said inducing of said promoter is performed at a temperature between 27 and 32 °C, preferably about 30 °C, most preferably 30 °C.

In a further specific embodiment said expression plasmid has the nucleotide sequence of SEQ ID NO:30, said major carbon source is glycerol, said feeding of said batch culture is performed with a flow rate, wherein said flow rate increases with an exponential coefficient $\mu$, and wherein said exponential coefficient $\mu$ is below $\mu_{max}$, said inducing of said promoter by co-feeding said batch culture is performed with a constant flow rate, wherein lactose and said major carbon source are co-fed to the batch culture in a ratio of about 2:1 to 1:3 (w/w), preferably about 1:1 (w/w), and wherein said cultivating and feeding of said batch culture and said inducing of said promoter is performed at a temperature between 27 and 32 °C, preferably about 30 °C, most preferably 30 °C.

DESCRIPTION OF THE FIGURES

Figure 1: Fermentation profile with pTac-nSD-Qb-mut (SEQ ID NO:1) in RB791 in 2 1 culture. The arrow indicates induction start 20% glycerol and 20% lactose. Shown are pump speed of feeding pump [%] (lower curve without symbols); glycerol concentration [g/l] (squares); lactose concentration [g/l] (diamond); oxygen partial pressure [%] (upper curve without symbols); β-Gal activity [U/ml*OD=1] (triangles with tip to the bottom); acetate concentration [g/l] (triangles with tip to the top); and OD600 (circles) plotted against the process time [h].

DETAILED DESCRIPTION OF THE INVENTION

Unless defined otherwise, all technical and scientific terms used herein have the same meanings as commonly understood by one of ordinary skill in the art to which this invention belongs.

"about": within the meaning of the present application the expression about shall have the meaning of+/-10 %. For example about 100 shall mean 90 to 110.

"promoter which is inducible by lactose" as used herein refers to a promoter which comprises regulatory elements of the lac operon. Such promoters are repressed by lacI and can be induced by IPTG or lactose. The skilled person is aware that induction of a promoter by lactose requires β-galactosidase activity in the bacterial host.

"located directly 3' ": a nucleotide sequence N2 which is located directly 3' of another nucleotide sequence N1 refers to a continuous sequence having the conformation 5'-N1-N2-3' wherein N1 and N2 are directly connected and not separated by additional sequence elements.

"sequence stretch": as used herein the term "sequence stretch" refers to a continuous nucleotide sequence which consists of less than 50, preferably less than 20, more preferably less than 10, even more preferably less than 5 nucleotides. In a further preferred embodiment the sequence stretch comprises or alternatively consists of at least one, preferably one, TAA stop codon. In another embodiment the sequence stretch comprises or alternatively consists of at least one, preferably one, TAA and at least one, preferably one, TGA stop codon. In further preferred embodiment the sequence stretch comprises or alternatively consists of SEQ ID NO:32.

"Batch culture" as used herein generally related to a culture of an organism in a culture medium in a closed system. Therefore, in contrast to a continuous culture, the density of the organism in the batch culture continuously increases with progressing cultivation time. The batch culture may be supplied with additional medium during the cultivation time (fed batch).

"Major carbon source" as used herein refers to the compound in the culture medium which contributes most to the carbon and energy supply of the bacterial host during the growth phase. It is interchangeably used with the term "substrate". The major carbon source is typically a sugar such as sucrose or glucose, or glycerol, and preferably glucose or glycerol.

"Conditions under which the promoter is repressed": it is to be understood that the repression of a promoter is an equilibrium of formation and dissociation of the repressor-operator complex and that even stringently repressed promoters may show a certain expression rate also in the absence of their inducer. Therefore, as used within the application the term "conditions under which the promoter is repressed" relates to conditions, wherein at the end of the growth phase, i.e. directly before the addition of lactose to the culture, the recombinant capsid protein is expressed to a level which does not exceed a concentration in the medium of 200 mg/l, preferably 150 mg/l, more preferably 100 mg/l, as determined by HPLC analysis (Example 17). Most preferably the recombinant protein is below the detection level.

"Inducer": within the meaning of the in invention the term "inducer" relates to any substance which directly or indirectly interacts with an inducible promoter and thereby facilitates expression from said promoter; for example, inducers of "a promoter inducible by lactose", such as the lac or tac promoter, are IPTG, lactose and allolactose.

"Coat protein" / "capsid protein": The term "coat protein" and the interchangeably used term "capsid protein" within this application, refers to a viral protein, preferably a subunit of a natural capsid of a virus, preferably of a RNA-phage, which is capable of being incorporated into a virus capsid or a VLP. For example, the specific gene product of the coat protein gene of RNA-phage Qβ is referred to as "Qβ CP", whereas the "coat proteins" or "capsid proteins" of bacteriophage Qβ comprise the "Qβ CP" as well as the A1 protein.

Recombinant capsid protein: A capsid protein which is synthesised by a recombinant host cell.

Polypeptide: As used herein the term "polypeptide" refers to a polymer composed of amino acid residues, generally natural amino acid residues, linked together through peptide bonds. Although a polypeptide may not necessarily be limited in size, the term polypeptide is often used in conjunction with peptide of a size of about ten to about 50 amino acids.

Protein: As used herein, the term protein refers to a polypeptide generally of a size of above 20, more particularly of above 50 amino acid residues. Proteins generally have a defined three dimensional structure although they do not necessarily need to, and are often referred to as folded, in opposition to peptides and polypeptides which often do not possess a defined three-dimensional structure, but rather can adopt a large number of different conformations, and are referred to as unfolded. The defined three-dimensional structures of proteins is especially important for the association between the core particle and the antigen, mediated by the second attachment site, and in particular by way of chemical cross-linking between the first and second attachment site using a chemical cross-linker. The amino acid linker is also intimately related to the structural properties of proteins in some aspects of the invention.

Recombinant host cell: As used herein, the term "recombinant host cell" refers to a host cell into which one ore more nucleic acid molecules of the invention have been introduced.

Recombinant VLP: The term "recombinant VLP", as used herein, refers to a VLP that is obtained by a process which comprises at least one step of recombinant DNA technology. The term "VLP recombinantly produced", as used herein, refers to a VLP that is obtained by a process which comprises at least one step of recombinant DNA technology. Thus, the terms "recombinant VLP" and "VLP recombinantly produced" are interchangeably used herein and should have the identical meaning.

RNA-bacteriophage: As used herein, the terms "RNA-bacteriophage" or "RNA-phage" refer to RNA viruses infecting bacteria, preferably to single-stranded positive-sense RNA viruses infecting bacteria.

Virus-like particle (VLP): as used herein, the term "virus-like particle" refers to a structure resembling a virus particle or it refers to a non-replicative or non-infectious, preferably a non-replicative and non-infectious virus particle, or it refers to a non-replicative or non-infectious, preferably a non-replicative and non-infectious structure resembling a virus particle, preferably a capsid of a virus. The term "non-replicative", as used herein, refers to being incapable of replicating the genome comprised by the VLP. The term "non-infectious", as used herein, refers to being incapable of entering the host cell. Preferably a virus-like particle in accordance with the invention is non-replicative and/or non-infectious since it lacks all or part of the viral genome or genome function. Typically a virus-like particle lacks all or part of the replicative and infectious components of the viral genome. A virus-like particle in accordance with the invention may contain nucleic acid distinct from their genome. A typical and preferred embodiment of a virus-like particle in accordance with the present invention is a viral capsid such as the viral capsid of the corresponding virus, bacteriophage, preferably RNA-phage. The terms "viral capsid" or "capsid", refer to a macromolecular assembly composed of viral protein subunits. Typically, there are 60, 120, 180, 240, 300, 360 and more than 360 viral protein subunits. Typically and preferably, the interactions of these subunits lead to the formation of viral capsid or viral-capsid like structure with an inherent repetitive organization, wherein said structure is, typically, spherical or tubular. For example, the capsids of RNA-phages or HBcAgs have a spherical form of icosahedral symmetry.

Virus-like particle of a RNA phage: As used herein, the term "virus-like particle of a RNA phage" refers to a virus-like particle comprising, or preferably consisting essentially of or consisting of coat proteins, mutants or fragments thereof, of a RNA phage. In addition, virus-like particle of a RNA phage resembling the structure of a RNA phage, being non replicative and/or non-infectious, and lacking at least the gene or genes encoding for the replication machinery of the RNA phage, and typically also lacking the gene or genes encoding the protein or proteins responsible for viral attachment to or entry into the host. Preferred VLPs derived from RNA-phages exhibit icosahedral symmetry and consist of 180 subunits. Within this present disclosure the term "subunit" and "monomer" are interexchangeably and equivalently used within this context. In this application, the term "RNA-phage" and the term "RNA-bacteriophage" are interchangeably used. A preferred method to render a virus-like particle of a RNA phage non replicative and/or non-infectious is by genetic manipulation.

one, a, or an: When the terms "one," "a," or "an" are used in this disclosure, they mean "at least one" or "one or more," unless otherwise indicated.

Sequence identity: The amino acid sequence identity of polypeptides can be determined conventionally using known computer programs such as the Bestfit program. When using Bestfit or any other sequence alignment program, preferably using Bestfit, to determine whether a particular sequence is, for instance, 95% identical to a reference amino acid sequence, the parameters are set such that the percentage of identity is calculated over the full length of the reference amino acid sequence and that gaps in homology of up to 5% of the total number of amino acid residues in the reference sequence are allowed. This aforementioned method in determining the percentage of identity between polypeptides is applicable to all proteins, polypeptides or a fragment thereof disclosed in this invention.

Sequence homology: The homology of nucleotide sequences can for example be determined by the program blastn which is an implementation of the BLAST algorithm, preferably using the default settings of the software.

"Fragment of a protein", in particular fragment of a recombinant protein or recombinant coat protein, as used herein, is defined as a polypeptide, which is of at least 70%, preferably at least 80%, more preferably at least 90%, even more preferably at least 95% the length of the wild-type recombinant protein, or coat protein, respectively and which preferably retains the capability of forming VLP. Preferably the fragment is obtained by at least one internal deletion, at least one truncation or at least one combination thereof. The term "fragment of a recombinant protein" or "fragment of a coat protein" shall further encompass polypeptide, which has at least 80%, preferably 90%, even more preferably 95% amino acid sequence identity with the "fragment of a recombinant protein" or "fragment of a coat protein", respectively, as defined above and which is preferably capable of assembling into a virus-like particle.

The term "mutant recombinant protein" or the term "mutant of a recombinant protein" as interchangeably used in this invention, or the term "mutant coat protein" or the term "mutant of a coat protein", as interchangeably used in this invention, refers to a polypeptide having an amino acid sequence derived from the wild type recombinant protein, or coat protein, respectively, wherein the amino acid sequence is at least 80%, preferably at least 85%, 90%, 95%, 97%, or 99% identical to the wild type sequence and preferably retains the ability to assemble into a VLP.

The invention is related to an efficient fermentation process for the production of a VLP of a bacteriophage. The process is improved with respect to yield of the VLP and can be up-scaled to a commercial production scale. The process encompasses the expression of recombinant capsid protein of bacteriophages in a bacterial host under conditions which allow the capsid protein to self-assemble into VLPs spontaneously.

Specific examples of VLPs which can be produced by the process of the invention are VLPs of bacteriophages, preferably RNA phages. In one preferred embodiment of the invention, the virus-like particle of the invention comprises, consists essentially of, or alternatively consists of, recombinant coat proteins, mutants or fragments thereof, of a RNA-phage. Preferably, the RNA-phage is selected from the group consisting of a) bacteriophage Qβ; b) bacteriophage R17; c) bacteriophage fr; d) bacteriophage GA; e) bacteriophage SP; f) bacteriophage MS2; g) bacteriophage M11; h) bacteriophage MX1; i) bacteriophage NL95; k) bacteriophage f2; 1) bacteriophage PP7 and m) bacteriophage AP205.

In one preferred embodiment of the invention, VLPs are produced comprising coat protein, mutants or fragments thereof, of RNA phages, wherein the coat protein has an amino acid sequence selected from the group consisting of: (a) SEQ ID NO:5. referring to Qβ CP; (b) a mixture of SEQ ID NO:5 and SEQ ID NO:15 (Qβ A1 protein); (c) SEQ ID NO:16 (R17 capsid protein); (d) SEQ ID NO:17 (fr capsid protein); (e) SEQ ID NO:18 (GA capsid protein); (f) SEQ ID NO: 19 (SP capsid protein); (g) a mixture of SEQ ID NO: 19 and SEQ ID NO:20; (h) SEQ ID NO:21 (MS2 capsid protein); (i) SEQ ID NO:22 (M11 capsid protein); (j) SEQ ID NO:23 (MX1 capsid protein); (k) SEQ ID NO:24 (NL95 capsid protein); (1) SEQ ID NO:25 (f2 capsid protein); (m) SEQ ID NO:26 (PP7 capsid protein); and (n) SEQ ID NO:12 (AP205 capsid protein).

Upon expression in E. coli, the N-terminal methionine of Qβ coat protein is usually removed (Stoll, E. et al., J. Biol. Chem. 252:990-993 (1977)). VLP composed of Qβ coat proteins where the N-terminal methionine has not been removed, or VLPs comprising a mixture of Qβ coat proteins where the N-terminal methionine is either cleaved or present are also within the scope of the present invention.

In one preferred embodiment of the invention, the VLP is a mosaic VLP comprising or alternatively consisting of more than one amino acid sequence, preferably two amino acid sequences, of coat proteins, mutants or fragments thereof, of a RNA phage.

In one very preferred embodiment, the VLP comprises or alternatively consists of two different coat proteins of a RNA phage, said two coat proteins have an amino acid sequence of SEQ ID NO: 5 and SEQ ID NO:15, or of SEQ ID NO:19 and SEQ ID NO:20.

In preferred embodiments of the present invention, the produced VLP comprises, or alternatively consists essentially of, or alternatively consists of recombinant coat proteins, mutants or fragments thereof, of the RNA-bacteriophage Qβ, fr, AP205 or GA.

In one preferred embodiment, the VLP is a VLP of RNA-phage Qβ. The capsid or virus-like particle of Qβ shows an icosahedral phage-like capsid structure with a diameter of 25 nm and T=3 quasi symmetry. The capsid contains 180 copies of the coat protein, which are linked in covalent pentamers and hexamers by disulfide bridges (Golmohammadi, R. et al., Structure 4:543-5554 (1996)), leading to a remarkable stability of the Qβ capsid. Capsids or VLPs made from recombinant Qβ coat protein may contain, however, subunits not linked via disulfide bonds to other subunits within the capsid, or incompletely linked. The capsid or VLP of Qβ shows unusual resistance to organic solvents and denaturing agents. Surprisingly, we have observed that DMSO and acetonitrile concentrations as high as 30%, and guanidinium concentrations as high as 1 M do not affect the stability of the capsid. The high stability of the capsid or VLP of Qβ is an advantageous feature, in particular, for its use in immunization and vaccination of mammals and humans.

Further preferred virus-like particles of RNA-phages, in particular of Qβ and fr in accordance of this invention are disclosed in WO 02/056905, the disclosure of which is herewith incorporated by reference in its entirety. Particular Example 18 of WO 02/056905 gave detailed description of preparation of VLP particles from Qβ.

In another preferred embodiment, the VLP is a VLP of RNA phage AP205. Assembly-competent mutant forms of AP205 VLPs, including AP205 coat protein with the substitution of proline at amino acid 5 to threonine, may also be used in the practice of the invention and leads to other preferred embodiments of the invention. WO 2004/007538 describes, in particular in Example 1 and Example 2, how to obtain VLP comprising AP205 coat proteins, and hereby in particular the expression and the purification thereto. WO 2004/007538 is incorporated herein by way of reference.

In one preferred embodiment, the VLP comprises or consists of a mutant coat protein of a virus, preferably a RNA phage, wherein the mutant coat protein has been modified by removal of at least one lysine residue by way of substitution and/or by way of deletion. In another preferred embodiment, the VLP of the invention comprises or consists of a mutant coat protein of a virus, preferably a RNA phage, wherein the mutant coat protein has been modified by addition of at least one lysine residue by way of substitution and/or by way of insertion. The deletion, substitution or addition of at least one lysine residue allows varying the degree of coupling with an antigene.

VLPs or capsids of Qβ coat protein display a defined number of lysine residues on their surface, with a defined topology with three lysine residues pointing towards the interior of the capsid and interacting with the RNA, and four other lysine residues exposed to the exterior of the capsid. Preferably, the at least one first attachment site is a lysine residue, pointing to or being on the exterior of the VLP.

Qβ mutants, of which exposed lysine residues are replaced by arginines are also encompassed by the present invention. Preferably these mutant coat proteins comprise or alternatively consist of an amino acid sequence selected from the group of a) Qβ-240 (SEQ ID NO:7, Lys13→Arg); b) Qβ-243 (SEQ ID NO:8, Asn10→Lys); c) Qβ-250 (SEQ ID NO:9, Lys2→Arg); d) Qβ-251 (SEQ ID NO:10, Lys16→Arg); and e) Qβ-259 (SEQ ID NO:11, Lys2→Arg, Lys16→Arg). The construction, expression and purification of the above indicated Qβ mutant coat proteins, mutant Qβ coat protein VLPs and capsids, respectively, are described in WO02/056905. In particular is hereby referred to Example 18 of above mentioned application.

In a further preferred embodiment the expressed protein is a capsid protein of bacteriophage AP205 having the amino acid sequence depicted in SEQ ID NO:12 or a mutation thereof, which is capable of forming a VLP, for example the proteins AP205P5T (SEQ ID NO:13) or AP205 N14D (SEQ ID NO:14.).

In a very preferred embodiment a VLP is produced which is composed of the 133 amino acid coat protein C of *E. coli* RNA phage Qβ having the amino acid sequence which is depicted in SEQ ID NO:5.

In a preferred embodiment, the nucleotide sequence encoding the recombinant capsid protein comprises at its 3' end a stop codon in addition to the naturally occurring stop codon to prevent read through. For example, plasmid pTac-nSDAP205 (SEQ ID NO:30) comprises an additional TGA stop codon directly 3' of the naturally occurring TAA stop codon.

The region of Qβ gene C corresponds to the NCBI GenBank Acc. No. M99039 (nucleotides 46-1062). Gene C contains sequences coding for both, the 133-amino acid Qβ coat protein (SEQ ID NO:5) and the 329-amino acid read through protein A1 (SEQ ID NO:15). In a preferred embodiment, the expression construct for the expression of Qβ CP or of a mutant or variant thereof comprises both, the nucleotide sequence encoding the Qβ CP and a nucleotide sequence encoding the Qβ A1 protein or a mutant or fragment thereof, wherein the nucleotide sequence encoding the Qβ A1 protein or the mutant or fragnent thereof is located directly 3' of the nucleotide sequendce encoding Qβ CP and wherein the expression of the Qβ A1 protein or mutant or fragment thereof is prevented by the presence of a TAA stop codon which is located between both sequences. In a preferred embodiment the naturally occurring leaky TGA stop codon is mutated to the stronger TAA stop codon (Kozlovska et al. 1996, Intervirology 39:9-15). Alternatively, and more preferrably, nucleotides 445-450 of NCBI Gen Bank Acc. No. M99039 (TGAACA, SEQ ID NO:31) are replaced by the sequence TAATGA (SEQ ID NO:32). For example, nucleotides 1-399 of SEQ ID NO:6 correspond to the coding region of the 133-amino acid Qβ CP (nucleotides 46- 444 of NCBI GenBank Acc. No. M99039), nucleotides 400 to 402 of SEQ ID NO:6 correspond to the strong TAA stop codon and nucleotides 403 to 405 of SEQ ID NO:6 to the leaky TGA stop codon, which is followed by a sequence relating to Qβ A1. Surprisingly, it was found that the presence of the nucleotide sequence relating to A1 in the expression construct results in higher RNA stability and, thus, in improved yield of Qβ CP and VLP as compared to a construct wherein the A1 sequence is deleted.

The expression of a recombinant protein can significantly reduce the growth rate of the bacterial host due to toxic effects of the accumulating protein and due to the metabolic burden caused by the protein synthesis. In particular cell lysis and low plasmid retention may occur. Inducible promoters provide for the possibility to separate the growth phase from the production phase of a fermentation process. Inducible promoters are repressed by a repressor molecule during the growth phase of the bacterial host and are induced by exposing the bacterial host to inductive conditions during the production phase. Inducible promoters therefore allow the bacterial host to grow fast, preferably exponentially during the growth phase and to reach high cell densities. Thus, inducible promoters provide for high yield of the expression product at the end of the production phase. Therefore, the usage of inducible promoters for the expression of recombinant protein is preferred.

A well known example for an inducible promoter is the lac promoter which forms part of the lac operon and which can be induced by addition of lactose or the strong synthetic inducer isopropylthio-β-D-galactosid (IPTG) to the growth medium of the bacterial host. Donavan et al. 2000 (Can. J. Microbiol 46:532-541) report on an improved process for the expression of a monoclonal antibody fragment under the control of the lac promoter. Further examples of inducible promoters are provided in table 1 of Makrides 1996 (Microbiological Reviews, p. 512-538).

A typical drawback of expression systems based on inducible promoters is the "leakiness" of the promoter, meaning that the promoter is only insufficiently repressed and causes a certain expression rate of the recombinant protein during the growth phase. This typically leads to a reduced cell density or to plasmid instability and, as a consequence, to reduced yield of the recombinant protein Makrides 1996 (Microbiological Reviews, p. 512-538). An example of a promoter which is prone to insufficient repression is the VHb promoter which is repressed under high oxygen conditions and induced upon oxygen depletion.

For the purpose of the invention promoters are preferred which are stringently repressed. In one embodiment the promoter is repressed by the repressor lacI. Examples of such promoters are disclosed in Makrides 1996 (Microbiol. Rev. 60:512-538), Goldstein & Doi 1995 (Biotechnology Annual Review 1:105-128), Hannig & Makrides 1998 (TIBTECH 16:54-60) and Stevens 2000 (Structures 8, R177-R185). In a preferred embodiment the promoter is inducible by lactose, more preferably it is selected from the group consisting of lac, lacUV5, tac, trc, $P_{syn}$ lpp$^a$, lpp-lac, T7-lac, T3-lac, and T5-lac. Especially preferred for the purpose of the invention is the tac promoter (SEQ ID NO:2) or a mutation or variant thereof. Within the scope of the invention are mutants or truncated or deleted variants of the tac promoter having a sequence homology with SEQ ID NO:2 which is at least 50 %, 60 %, 70 %, 80, 90, or 95%, preferably 98 to 100 %, most preferably 99 %, wherein the promoter strength is comparable to that of the promoter of SEQ ID NO:2. The skilled person will be able to determine the promoter strength of a given sequence by comparative expression studies using standard methods. In a specific embodiment of the invention the promoter driving the expression of the recombinant capsid protein comprises or alternatively consists of SEQ ID NO:2. The tac promoter is a fusion product of the -10 region of the lacUV5 promoter and the -35 region of the trp promoter and combines the high transcription efficiency of trp with the regulatory elements of the lac promoter (de Boer et al. 1983, PNAS 80:21-25; Amann et al. 1983 Gene 25:167-178). It provides for sufficiently high expression rates and high protein yield while avoiding the formation of insoluble or incorrectly folded recombinant protein which may occur with stronger promoters, such as the T7 promoter. The trc and the tic promoter are mutated versions of the tac promoter (Brosius et al. 1985, The Journal of Biological Chemistry 260(6):3539-3541). In a further preferred embodiment the promoter is selected from the group consisting of tic, trc and tac.

For the construction of an expression construct for the purpose of the invention the promoter is operably linked to the nucleotide sequence encoding the recombinant capsid protein via a ribosome binding site (Shine-Dalgamo sequence, SD), typically comprising an ATG start codon at its 3' end. Suitable Shine-Dalgarno sequences for the purpose of the invention are well known in the art (Dalbøge et al. 1988, DNA 7(6):399-405; Ringquist et al. 1992, Mol. Micr. 6: 1219-1229). In one embodiment of the invention the expression construct comprises the SD sequence of Dalbøge et al. 1988 (DNA 7(6):399-405) which is depicted in SEQ ID NO:4. In another, preferred, embodiment the expression construct comprises a Shine-Dalgarno sequence of Ringquist et al. 1992 (Mol. Micr. 6:1219-1229, SEQ ID NO:3, nSD). Surprisingly, it was found that SEQ ID NO:3 is particularly suited for the purpose of the invention because it results in improved expression levels and improved yield of recombinant capsid protein. SEQ ID NO:3 is especially suited to enhance the expression of AP205 capsid protein.

Transcriptional terminators are indispensable elements of expression constructs. The skilled person will be able to choose a suitable terminator sequence form a wide range of sources. In a preferred embodiment of the invention the terminator sequence is the rRNB terminator sequence which is depicted in SEQ ID NO:28.

For the purpose of plasmid selection the skilled person will typically use an antibiotic resistance marker gene. Examples of antibiotic resistance genes which are widely used in the art and which are suitable for the purpose of the invention are resistance genes against the antibiotics ampicillin, tetracyclin and kanamycin. The use of kanamycin as a selective agent in the frame of a process for the production of a VLP is generally preferred because of the lower allergenic potential of kanamycin as compared to alternative antibiotics and because of the lower safety concerns resulting thereof for the use of the VLP as a vaccine. Furthermore, kanamycin provides better plasmid retention as compared to alternative antibiotics such as ampicillin. The kanamycin 3'-phosphotransferase gene (SEQ ID NO:29) which is derived from the transposon Tn903 is therefore a particularly useful selectable marker gene.

The addition of antibiotics to the medium is generally undesirable in a commercial production process for cost and safety reasons. For the purpose of the invention, antibiotics, preferably kanamycin, are only used for the selection of the expression strain. The culture media of the fermentation process are essentially free of antibiotics, in particular kanamycin. However, addition of an antibiotic to the preculture which is used to produce the inoculum for the main culture can improve plasmid retention throughout the process (Example 10).

The skilled person will create expression plasmids which are useful for the production of VLPs of bacteriophages by combining the genetic elements described above applying standard methods of molecular biology. Particularly useful expression constructs for the purpose of the invention are pTac-nSDQb-mut (SEQ ID NO: 1) for the production of Qβ VLP and pTac-nSDAP205 (SEQ ID NO:30) for the production of AP205 VLP. The construction of these specific expression plasmids is described in detail in the Examples section.

The expression plasmids are transformed to a bacterial expression host by electroporation or any other transformation method known in the art. Individual clones of the host comprising the expression plasmid are selected for maximal expression of the recombinant capsid protein by SDS-PAGE after cell lysis. Selected clones of the expression host comprising the expression plasmid can be stored as frozen glycerol cultures.

Escherichia coli strains having the specific features described in the following sections are preferred expression hosts for the invention.

In a preferred embodiment the repression of the promoter is improved by overexpression of the repressor by the bacterial host, wherein the gene causing the overexpression may be located on the chromosome of the host cell or on a separate plasmid, preferably a high copy number plasmid. Alternatively, the gene causing overexpression of the repressor is located on the plasmid carrying the expression construct. Examples of genes causing overexpression of lacI are $lacI^q$ (Menzella et al. 2003, Biotechnology and Bioengineering 82(7)809-817) which is a single CG to TA change at -35 of the promoter region of lacI which causes a 10 fold increase in LacI expression. A further example is lacIQ1 (Glascock & Weickert 1998, Gene 223(1-2):221-231). Improved repression of the promoter during the growth phase results in improved plasmid retention and higher cell density and, ultimately, in improved protein yield. For example, bacterial strains comprising the $lacI^q$ gene overexpress the lacI repressor molecule and therefore prevent formation of the recombinant protein during the growth phase more efficiently than strains comprising the wildtype gene. In a specifically preferred embodiment the bacterial host comprises the $lacI^q$ gene on its chromosome.

Upon exposure of the bacterial host to an inducer substance, the repressor is inactivated and the promoter becomes active. Addition of the strong inducer IPTG to the culture medium results in an immediate increase of the expression rate of the recombinant protein to a high level because IPTG directly enters the cells by diffusion and binds and inactivates the active repressor lacI. Inactivated lacI repressor molecules dissociates from the operator and allow high level transcription from the promoter. IPTG is not metabolized by the cell and the transcription continues with high rates until other metabolic parameters become limiting.

As mentioned before, high expression rates may lead to the formation of insoluble recombinant protein which is not capable of forming a VLP by self-assembly. Induction of protein expression with high concentrations of IPTG is particularly prone to the formation of insoluble protein. Therefore, induction of the promoter is preferably achieved by the addition of IPTG in concentrations which are below the concentration which causes the expression to occur at its maximum rate (Kopetzki et al. 1989, Mol Gen Genet 216:149-155). In one embodiment of the invention induction of the promoter is achieved with IPTG, wherein the concentration of IPTG in the culture of the bacterial host is about 0.001 to 5 mM, preferably 0.001 to 1 mM, more preferably 0.005 to 1 mM, still more preferably 0.005 to 0.5 mM. In a specifically preferred embodiment the concentration of IPTG is about 0.01 mM, most preferrably 0.01 mM.

Alternatively, induction of the promoter is achieved by the addition of lactose. Induction of recombinant protein expression with lactose requires that the bacterial host is capable of taking up lactose from the medium, e.g. by Lac permease and that it comprises β-galactosidase activity. The Lac permease dependent uptake of lactose into the cells follows a slower kinetic than the uptake of IPTG by diffusion. Furthermore, lactose does not directly interact with the lac operon but is converted by β-galactosidase to allolactose (1-6-O-β-galactopyranosyl-D-glucose) which is the actual inducer of the promoter. Induction of recombinant protein expression by the addition of lactose is advantageous because it avoids the immediate increase of the expression rate to a maximum level upon addition of the inducer and, thus, it reduces the risk of the formation of insoluble protein.

Allolactose is metabolised by the bacterial host during the production phase and contributes carbon and metabolic energy to the bacterial metabolism. This may further contribute to improved protein yield as compared to induction with IPTG. Furthermore, induction by lactose allows to a certain extend the control of the expression rate of the recombinant protein during the production phase via the lactose concentration in the culture medium. Induction by lactose is further preferred in a pharmaceutical production process because IPTG is expensive and is believed to be toxic. Its removal needs to be demonstrated at the end of a the production process.

For the afore said reasons, the process of the invention comprises the expression of a recombinant capsid protein of a bacteriophage under the control of the tac promoter or mutant or variant thereof, in a bacterial host which comprises a gene, preferably $lacI^q$, causing overexpression of a repressor of the tac promoter. In case induction is performed with lactose, the bacterial host further is able to take up lactose from the medium and comprises β-galactosidase activity. Such bacteria strains can, for example, be obtained from strain collections such as ATTC (http://www.atcc.org). In a preferred embodiment, the bacterial strain is an E. coli strain, preferably an E. coli strain selected from the group consisting of RB791, DH20, Y1088, W3110 and MG1655. Most preferably, the bacterial strain is E. coli RB791.

The pH of the culture medium of the bacterial host can be controlled during the fermentation process and regulated by the addition of acidic or alkaline solutions using methods which are well known in the art. Within the physiological range, the pH of the medium is not a critical parameter. The pH of the medium is preferably between 5.5 and 8.0, more preferably between 6.5 and 7.5, even more preferably between 6.7 and 7.0 and most preferably the pH of the medium is 6.8. The pH of the culture medium may be kept constant during the fermentation process or it may follow a certain profile during the different phases of the process within the pH ranges specified above. In a preferred embodiment the pH is kept constant at a value of 6.7 to 7.0, preferably it is kept constant at 6.8.

The oxidative capacity of bacteria cells is limited and high concentrations of the substrate may cause the formation of reduced products like acetate, which may lead to undesired acidification of the medium and to reduced growth of the bacteria. Therefore, the bacterial host is grown in a fed-batch culture on a minimum medium with a limited quantity of the carbon source (substrate) and further supplied with a limited quantity of the substrate to avoid the formation of undesired metabolic products like acetate.

In one embodiment of the invention the growth phase of the fermentation process is divided in a batch phase and a feed phase. The process begins with a batch phase in growth medium, preferably a minimal medium, in which the substrate (major carbon source) supplied with the medium is consumed. Once the substrate is almost exhausted, medium containing the substrate is fed to the culture at the same rate as the desired growth rate of the host cell (feed phase), i.e. the growth rate of the bacterial host is limited by the feed rate of the substrate. It is understood by the skilled person that the decisive parameter is the actual mass flow of the substrate and other nutrients required to maintain growth. Since in practice a constant composition of the feed medium can be assumed, the flow rate refers to the volume flow of the medium. The same consideration applies to the co-feed medium (see below).

During the feed phase the growth rate can be freely selected in a wide range nearly up to the maximum growth rate ($\mu_{max}$) if no inhibition occurs. The actual value of $\mu_{max}$ is highly dependent on the bacterial strain, the expression construct and the growth conditions. The skilled person will understand that the determination of $\mu_{max}$ is performed under conditions under which the promoter is repressed.

For a given experimental set up, $\mu$ can be determined from the growth curve of the culture by plotting biomass concentration (x) as determined by $OD_{600}$ or cell wet weight (CWW) against the cultivation time and determining the exponential growth coefficient $\mu$ based on the equation $x = x_0 e^{\mu t}$. The actual value of $\mu_{max}$ is determined as the growth rate $\mu$ of an exponentially growing batch culture in the beginning of the batch phase when no substrate limitation occurs, i.e. without supply of additional medium by feeding. The growth rate $\mu$ can be determined by computing the ratio of the difference between natural logarithm of the total biomass $X_2$ measured at time $t_2$ and natural logarithm of the total biomass $X_1$ measured at time $t_1$ to the time difference $(t_2 - t_1)$: $\mu = (\ln X_2 - \ln X_1) / (t_2 - t_1)$.

Fed-batch culture allows the maintenance of a constant growth rate ($\mu$). In a preferred embodiment the substrate is fed during the feed phase according to the exponential increase of the biomass (x). If during the feed phase the substrate is supplied at the same rate it is consumed, the culture is in a quasi steady state, analogous to the cultivation in a continuous culture. Because biomass formation and substrate consumption are correlated over the substrate-referred yield coefficient $Y_{x/s}$ (biomass [g] / substrate [g]), the substrate quantity (s) per time unit (t) to be supplied is calculated according to the formula $ds/dt = \mu/Y_{x/s}\, x_{0\,tot}\, e^{\mu t}$, wherein $x_{0\,tot}$ is the total biomass at feed start. In a further preferred embodiment the growth rate $\mu$ of the bacterial host during the feed phase of the process is set to a value which is below $\mu_{max}$. More preferably, $\mu$ is about 30 % to 70 %, most preferably about 50 % of $\mu_{max}$. In a specific embodiment of the invention $\mu$ is set to an absolute value of 0.15 to 0.45 $h^{-1}$, more preferably 0.25 to 0.35 $h^{-1}$, most preferably $\mu$ is 0.3 $h^{-1}$, provided that the set up of the process is such, that these values are below $\mu_{max}$.

Bacteria are able to utilise a wide range of different substrates. For the purpose of the invention, preferred major carbon sources are glucose and glycerol, preferably glycerol. Although the maximum specific growth rate ($\mu_{max}$) of the expression host which can be achieved may be higher with glucose than with glycerol, glycerol causes less acetate formation and provides higher biomass yield per substrate ($Y_{x/s}$) and, ultimately, higher yield of the recombinant protein. Furthermore, the handling of the liquid substrate glycerol is easier than that of solid carbon sources like glucose which need to be dissolved in a separate process step.

As mentioned before, plasmid retention, i.e. the maintenance of the expression plasmid in the bacterial host during the fermentation process, is essential for optimal yield of the recombinant protein. Plasmid retention can be assessed by spreading bacteria cells on a solid medium to form single colonies and testing individual colonies for their antibiotic resistance. For example, a plasmid retention of 100 % means that 100 out of 100 tested colonies comprise the specific antibiotic resistance which conferred by the expression plasmid. For the purpose of the invention plasmid retention at the end of the fermentation process is more than 80 %, preferably more than 90 %, more preferably more than 95 %, even more preferably more than 97 % and most preferably 100 %.

The optimal growth temperature of a bacterial strain is the temperature at which it reaches its highest maximal growth rate ($\mu_{max}$). Under otherwise not limiting conditions for most E. coli strains this temperature is about 37 °C. However, growth of the bacterial strain comprising the expression construct at the optimal growth temperature and in the absence of a selective antibiotic may favour the loss of the expression plasmid, whereas plasmid retention is generally improved when the expression strain is grown at lower temperature. Although the maximum growth rate of the expression strain is lower when the strain is grown at temperatures below its optimal growth temperature as compared to growth at the optimal growth temperature, the yield of recombinant protein may be equal or even better at the lower temperature due to improved plasmid retention. In one embodiment of the invention the bacterial host is therefore cultivated at a temperature below its optimal growth temperature. In a preferred embodiment the growth temperature is between 20 and 37 °C, preferably between 23 and 35 °C, more preferably between 25 and 33 °C, even more preferably between 27 and 32 °C, still more preferably between 28 and 31 °C. Even more preferred is a temperature of about 30 °C, most preferably 30 °C.

The growth of the bacterial host during the fermentation process can be assessed by determining the optical density at 600 nm ($OD_{600}$), the cell wet weight (CWW [g/l]) and the cell dry weight (CDW [g/l]). These parameters can be used to define the optimal time point for the start of the production phase by addition of the inducer, preferably lactose, to the medium. It is apparent for the skilled person, that on one hand higher CWW at the induction can be achieved by an extended feed-phase and may lead to improved yield of the recombinant protein but that on the other hand over-aged cultures may show insufficient protein expression. The optimal time point for the expression start therefore needs to be determined for the specific production conditions. For example, for expression of Qβ CP in E. coli RB791 in a total volume of 2 1, induction is started after ca. 14 h, when $OD_{600}$ has reached about 40 to 60. For the same process in a 50 l scale induction start is also after ca. 14 h when $OD_{600}$ has reached about 50.

In one embodiment of the invention induction is started when the $OD_{600}$ reached a value of 25 to 60, preferably 25 to 55, more preferably 30 to 50, most preferably 30 to 40. In a specifically preferred embodiment induction is started when $OD_{600}$ is 35.

In one embodiment of the invention induction is achieved by the addition of IPTG as described above, wherein preferably feeding of the culture with the major carbon source is continued. Since IPTG is not metabolized by the bacterial host, induction can be achieve by a single addition of IPTG to the desired concentration. Alternatively, induction can be achieved by a continuous flow of IPTG to the culture. In a prefered embodiment induction is performed by addition of IPTG in a single addition or a continuous flow, wherein said feeding of the batch culture with the major carbon source is continued with a constant or an exponentially increasing flow rate of the major carbon source.

As described above, the induction of protein expression can alternatively be achieved by the addition of lactose to the culture medium. In one embodiment of the invention, at the induction start the exponential feed of the substrate is interrupted and the culture is supplied with a constant flow of induction medium containing 100 to 300 g/l, preferably 100 g/l lactose as the sole carbon source (lactose feed medium). Preferably, the constant flow rate of lactose equals approximately the flow rate of the substrate at the end of the feed phase.

Upon addition of lactose to the culture, the β-galactosidase activity increases, lactose is converted to allolactose which induces the tac promoter and the expression of the recombinant capsid is initiated. In parallel, allolactose is further metabolised and contributes to the energy supply for the bacterial host. The equilibrium of the feeding rate of the induction medium and the lactose consumption by the cells thus determines the expression rate. The enzymatic reactions involved in this cascade allow to control the process in such a way that the formation of inclusion bodies is minimised. The progress of induction process can be monitored by determining the β-galactosidase activity in the culture, e.g. by a β-Gal Assay Kit (Invitrogen, K1455-01).

The expression of the recombinant protein is an energy demanding process. To prevent yield loss which might be caused by the excessive consumption of the inducer by the bacterial host and low expression rates resulting thereof, the culture can be additionally supplemented with substrate (major carbon source) during the production phase. In one embodiment the ratio between lactose and substrate, preferably glycerol, in the induction medium ranges from 2:1 to 1:

3 (w/w). In a preferred embodiment the ratio of lactose and substrate, preferably glycerol, is 1:1 (w/w). In a more preferred embodiment the induction medium comprises ca. 200 g/l lactose and ca. 200 g/l glycerol. Preferably, the constant flow rate of the substrate, preferably glycerol, during co-feed equals approximately the flow rate of the substrate at the end of the feed phase.

The growth of the bacterial host as determined by CDW, CWW or $OD_{600}$ continues during the production phase at a growth rate which is lower than that during the growth phase and which is decreasing with the process time. In a further embodiment of the invention co-feed of inducer, preferably lactose and glycerol during the production phase is performed with an increasing flow rate, preferably with a flow rate wherein the incremental increase of the flow rate is adapted to the actual growth rate of the culture. In a preferred embodiment, the ratio between lactose and glycerol in the induction medium ranges from 2:1 to 1:3 (w/w). In a more preferred embodiment, the ratio of lactose and glycerol is 1:1 (w/w). In an even more preferred embodiment the induction medium comprises ca. 200 g/l lactose and ca. 200 g/l glycerol.

In one embodiment of the invention induction is performed by co-feeding the inducer, preferably lactose and the major carbon source to the culture, wherein the inducer and the major carbon source are contained in separate media which are separately fed to the culture. In a preferred embodiment the inducer, preferably lactose and the major carbon source are contained in the same medium (co-feed medium).

At the end of the production phase the cells are harvested by centrifugation. Typically, cells are harvested about 5 h after induction start, when a final $OD_{600}$ of 90 to 130 is reached. Further extension of the production phase may lead to higher $OD_{600}$ and CWW values and therefore to further improved yield of the expression construct.

Harvested cells may be suspended in a storage buffer and stored at -80 °C for further processing.

The total protein content of the cells is determined after cell lysis by SDS PAGE and comparison with a protein standard. The content of soluble protein is determined by HPLC. The identity of the expressed capsid protein is determined by western blotting. The concentration of assembled VLPs can be analysed by size exclusion chromatography (Example 18). VLP can preparatively be purified from lysed cells by chromatographic methods.

Upscale of the process of the invention to large volumes is possible with only minor adaptations. The invention encompasses culture volumes in the range of 100 ml up to 6000 1. Preferred culture volumes are 40 to 100 1, most preferably about 50 1. It is apparent for the skilled person that larger culture volumes in particular require larger volumes of the preculture which is used for inoculation. For example, a preculture may be performed in two ore more steps with increasing preculture volume. To ensure plasmid retention in large culture volumes, the precultures which are used as inoculum may contain an antibiotic to maintain selection pressure. The skilled person is aware that plasmid retention can further be improved by reducing the number of generations which is necessary to reach the desired final cell density. Therefore, it is advantageous to inoculate the precultures and the batch cultures with high cell densities. In a preferred embodiment the initial $OD_{600}$ of the preculture is 0.1 to 0.4, preferably about 0.3.

In one embodiment of the invention the inoculum for the batch culture is produced in a preculture process comprising the step of growing the bacterial host in a medium comprising an antibiotic, preferably kanamycin. More preferably, said preculture process comprises the steps of growing the bacterial host in a first medium comprising an antibiotic, preferably kanamycin, and diluting said first medium comprising the bacterial host with a second medium to an $OD_{600}$ of 0.1 to 0.4, preferably about 0.3, wherein said second medium is essentially free of an antibiotic, and and further cultivating said bacterial host.

EXAMPLES

Example 1

**Cloning Strategy for the Expression Plasmid pTac-nSD-Qb-mut (SEQ ID NO:1)**

The coat protein-encoding gene (C) of *E. coli* RNA phage Qβ is amplified from plasmid pSDQb-mut (SEQ ID NO:33). The plasmid contains the sequence of gene C coding for the 133-aa Qβ coat protein (CP) and the 329-aa read through protein (A1). To prevent read-through, nucleotides 445-450 according to NCBI GenBank Acc. No. M99030 TGAACA (SEQ ID NO:31) are replaced by the sequence TAATGA (SEQ ID NO:32).

The coat protein-encoding gene C from plasmid pSDQb-mut is amplified by PCR. Oligonucleotide Qb-FOR3/2 (SEQ ID NO:34) with an internal *Eco*RI site and a synthetic Shine-Dalgamo (SD, SEQ ID No:4) sequence anneals to the 5' end of the Qβ CP gene. Oligonucleotide Qblang-REV2/2 (SEQ ID NO:35) contains an internal *Hind*III site and primes to the 3' end of the noncoding region of gene C. The 1054 bp amplified PCR fragment includes nucleotides 46-1062 of NCBI GenBank Acc. No. M99039 (except the nucleotide changes described above) and the synthetic SD sequence. The PCR fragment is digested with the restriction enzymes *Hind*III/*Eco*RI and the resulting 1036 bp fragment is inserted into the *Hind*III/ *Eco*RI restriction sites of a modified pKK223-3 vector (Pharmacia, NCBI GenBank Acc. No.: M77749, SEQ ID NO:27). In this modified pKK223-3 vector the ampicillin resistance gene is replaced with the kanamycin resistance gene of vector pUC4K (Pharmacia, NCBI GenBank Acc. No.: X06404, SEQ ID NO:37).

Vector pTac-nSDQb-mut (SEQ ID NO:33) differs from vector pTacQb-mut in the Shine-Dalgamo sequence. This Shine-Dalgamo sequence (nSD, SEQ ID NO:3) is introduced by amplifying the Qβ coat protein-encoding gene C via PCR from plasmid pTacQb-mut. Oligonucleotide nSDQb-mut*Eco*RIfor (SEQ ID NO:36) with an internal *Eco*RI site and the corresponding synthetic Shine-Dalgarno (nSD) sequence anneals to the 5' end of the Qβ CP gene. Oligonucleotide Qblang-REV2/2 (SEQ ID NO:35) contains an internal *Hind*III site and primes to the 3' end of the noncoding region of gene C. The 1054 bp amplified PCR fragment includes nucleotides 46-1062 of NCBI GenBank Acc. No. M99039 (except the nucleotide changes described above) and the synthetic nSD sequence. The PCR fragment is digested with the restriction enzymes *Hind*III/ *Eco*RI and the resulting 1036 bp fragment is inserted into the *Hind*III/ *Eco*RI restriction sites of a modified pKK223-3 vector (Pharmacia, NCBI GenBank Acc. No.: M77749, SEQ ID NO:27). In this modified pKK223-3 vector the ampicillin resistance gene is replaced with the kanamycin resistance gene of vector pUC4K (Pharmacia, NCBI GenBank Acc. No.: X06404, SEQ ID NO:37).

Example 2

**Cloning Strategy for the Expression Plasmid pTac-nSD-AP205 (SEQ ID NO:30)**

The coat protein-encoding gene of *Acinetobacter* phage AP205 is amplified from plasmid pAP205-58. This plasmid contains the sequence of the coat protein gene (corresponding to nucleotides 1908-2303 of NCBI GenBank Acc. No. AF334111) coding for the 131-amino acid capsid protein of phage AP205.

The coat protein-encoding gene is amplified by PCR. Oligonucleotide nSDAP238-*Eco*RIfor (SEQ ID NO:38) with an internal *Eco*RI site and a synthetic Shine-Dalgamo (nSD) sequence anneals to the 5' end of the coat protein gene. Oligonucleotide AP238*Hind*IIIrev (SEQ ID NO:39) contains an internal *Hind*III site and primes to the 3' end of the coat protein gene. This oligonucleotid introduces a second stop codon behind the naturally occurring stop codon of the coat protein. The 438 bp amplified PCR fragment includes nucleotides 1908-2303 of NCBI GenBank Acc. No. AF334111 and the synthetic nSD sequence. The PCR fragment is digested with the restriction enzymes *Hind*III/ *Eco*RI and the resulting 420 bp fragment is inserted into the *Hind*III/ *Eco*RI restriction sites of a modified pKK223-3 vector (Pharmacia, NCBI GenBank Acc. No.: M77749, SEQ ID NO:27). In this modified pKK223-3 vector the ampicillin resistance gene is replaced with the kanamycin resistance gene of vector pUC4K (Pharmacia, NCBI GenBank Acc. No.: X06404, SEQ ID NO:37).

Example 3

**Expression of Qβ CP under control of the tac promoter and nSD**

The *E. coli* strain RB791 was transformed with plasmids pTac-nSD-Qb-mut (SEQ ID NO:1). The clone was grown in shake flasks. Each flask contained 100 ml of R40 medium (main culture medium, Hypep 7455, glycerol, see Example 5) with kanamycin (25μg/ml) and was inoculated with over night cultures at a start $OD_{600}$ of 0.3. The shake flasks were incubated for 4 h ($OD_{600}$ between 4 and 5) at 30 °C and an agitation of 220 rpm. The induction was carried out with 0.5 % of lactose for 4 h. Protein production was determined by SDS-PAGE. The gel showed a strong protein band which was identified as Qβ CP.

Example 4

**Expression of AP205 CP under control of the tac promoter and SD vs. nSD**

9 clones of pTac-nSDAP205 (SEQ ID NO:30) and 6 clones of pTac-SDAP205 were screened in shake flasks. pTac-SDAP205 (SEQ ID NO:40) is identical to pTac-nSDAP205 but comprises the Shine-Dalgamo sequence of SEQ ID NO:4 instead of that of SEQ ID NO:3. Each flask contained 50 ml of R40 medium (main culture medium, Hypep 7455, glycerol, see Example 5) with kanamycin (25 μg/ml) and was inoculated with over night cultures at a start $OD_{600}$ of 0.3 (for pTac-nSDAP205) or 0.4 (pTac-SDAP205). The shake flasks were incubated for 4 h at 30°C and an agitation of 220 rpm. The induction was carried out with 0.5% of lactose. Protein production was determined by SDS-PAGE. For all tested clones expression of AP205 CP was significantly stronger from pTac-nSDAP205 than from pTac-SDAP205.

Example 5

**Composition of Culture Media**

Culture media were composed as described in Table 1.

Table 1: Composition of Culture media.

| | Concentrations in [g/L] | | | | |
|---|---|---|---|---|---|
| | Main Medium + Hypep **R27** | Main Medium + Hypep + Glycerol **R40** | Feed Medium + 50% Glycerol **R41** | Induction Medium +20% Glycerol +20% Lactose **R42** | Main Medium + Bacto YE + Glycerol **R43** |
| Component | | | | | |
| $Na_2HPO_4 2H_2O$ | 2.5 | 2.5 | 2.5 | 2.5 | 2.5 |
| $KH_2PO_4$ | 3 | 3 | 3 | 3 | 3 |
| $K_2HPO_4$ | 5.2 | 5.2 | 5.2 | 5.2 | 5.2 |
| Citrate | 3.86 | 3.86 | 3.86 | 3.86 | 3.86 |
| $(NH_a)2SO_4$ | 4 | 4 | 4 | 4 | 4 |
| Vit B1 | 0.01 | 0.01 | 0.02 | 0.02 | 0.01 |
| $CaCl_2\ 2H_2O$ | 0.0147 | 0.0147 | 0.0147 | 0.0147 | 0.0147 |
| $MgSO_4\ 7H_2O$ | 0.5 | 0.5 | 9 | 9 | 0.5 |
| $FeCl_3\ 6H_2O$ | 0.054 | 0.054 | 0.054 | 0.054 | 0.054 |
| $CoCl_2\ 6H_2O$ | 0.0005 | 0.0005 | 0.0005 | 0.0005 | 0.0005 |
| $MnCl_2\ 4H_2O$ | 0.003 | 0.003 | 0.003 | 0.003 | 0.003 |
| $CuCl_2\ 2H_2O$ | 0.0003 | 0.0003 | 0.0003 | 0.0003 | 0.0003 |
| $H_3BO_3$ | 0.003 | 0.003 | 0.003 | 0.003 | 0.003 |
| $Na_2MoO_4 2H_2O$ | 0.0005 | 0.0005 | 0.0005 | 0.0005 | 0.0005 |
| Zn $(CH_3COO)_2 2H_2O$ | 0.0026 | 0.0026 | 0.0026 | 0.0026 | 0.0026 |
| Glucose | 5 | --- | --- | --- | --- |
| Glycerol | --- | 5 | 500 | 200 | 5 |
| Lactose anhydrous | --- | --- | --- | 200 | --- |
| HyPep 7455 | 5 | 5 | --- | --- | --- |
| Bacto Yeast Extract | --- | --- | --- | --- | 5 |

Example 6

**Expression of Qβ CP in a Fed-batch Process (2 L Scale)**

The fermentation process was performed in a bioreactor (Applikon 5 L dished bottom) equipped with 2 disc stirrer (Ø 6 cm), baffles (3x 16 cm), pH-, pO2-, and temperature control, and fermenter software BioXpert Version 2.22

5 μL cryo culture of RB791 transformed with plasmids pTac-nSD-Qb-mut were inoculated in 100 mL Erlenmeyer flasks containing 50 mL medium R40 (25μg/mL kanamycin) and cultivated for 14 h at 30°C and 220 RPM over night. After 14 h an $OD_{600}$ value of 6.0 was reached. For batch fermentation, 2 L of medium (R40) were pumped into the bioreactor. In Table 2 the cultivation parameters are listed.

Table 2: Parameter set points for batch phase.

| *Parameter* | *Set point* | *Unit* |
|---|---|---|
| Stirrer speed | 1000 | [rpm] |
| Air supply | 2.5 | [L/min] |

(continued)

| Parameter | Set point | Unit |
|---|---|---|
| 02-supply, maximal | 2 | [L/min] |
| Temperature | 30 | [°C] |
| O2-saturation | > 40 | [%] |
| pH | 6.8 | [-] |

The bioreactor was inoculated with 100 mL inoculum. Samples of 2 mL were taken, $OD_{600}$ determined and centrifuged at 14'000 RPM. Pellet and supernatant were separated and frozen for further analysis. The biomass concentration [g/L] was calculated using the following equation:

$$OD_{600} \text{ x } 0.45 \text{ [g x } L^{-1} \text{ x } OD_{600}^{-1}] = \text{biomass [g/L].}$$

The Qbeta content in per cent of the total protein content was calculated as follows, assuming, that 50 % of the *E. coli* biomass is protein:

$$\text{Biomass [g x } L^{-1}]/2 = \text{total protein [ g x } L^{-1} \text{ ]}$$

$$\text{Qbeta [g x } L^{-1}]/\text{total protein [ g x } L^{-1} \text{ ] x 100} = \text{Qbeta/total protein [\%].}$$

In the fed-batch mode, which followed the batch mode, a feeding phase was added. In the feeding phase substrate is supplied to the cells in the reactor according to a defined profile. The feed profile depends on the selected growth rate $\mu$, the yield coefficient biomass to glycerol ($Y_{x/glycerol}$), the volume (Vf), and the concentration of substrate in the feed (cf). substrate concentration. The feed was calculated using the following equation:

Feed equation

$$mf = (\mu / Y_{x/s} + m) \quad Vf \times Xf \times e^{\mu t}$$
$$pump = (mf / cf + b)/a$$

mf = mass flow [g/h]
$\mu$ = specific growth rate [1/h]
$Y_{x/Glycerol}$ = Yield biomass to glycerol [g/g]
m = maintenance energy [$g*g^{-1}*h^{-1}$]
Vf = Volume at feed start
Xf = Biomass at feed start
cf = Concentration of substrate in feed [g/mL]
a+b = offset / slope of pump calibration equation

For the determination of the calibration parameters a and b, a pump calibration was carried out. In addition, the feed tube with feed bottle was clamped into the feed pump and the pump was run with 7, 14 and 21 % pump performance. The pumped feed volume per time was noted. In a resulting diagram of the relation of pump performance [%] to pumped feed solution [mL/h], the slope (a) and the Y-axis section (b) was determined. On the bioreactor the parameters in Table 3 were set for fed-batch cultivation.

Table 3: Parameters for fed-batch cultivation in bioreactor.

| *Parameter* | *Set point* | *Unit* |
|---|---|---|
| Stirrer speed | 1000 | [rpm] |
| Air supply | 2.5 | [L/min] |
| $O_2$-supply, maximal | 2 | [L/min] |
| Temperature | 30 | [°C] |
| $O_2$-saturation | > 40 | [%] |
| pH | 6.8 | [-] |

After reaching a process time of approximately 7 h (end of batch) the feed pump was turned on automatically. After further 7 h cultivation, when the $OD_{600}$ reached 55-60, the feed medium (for biomass propagation) was exchanged with the induction medium R42 (for biomass propagation and induction). After 5 h feeding of R42 was stopped and the culture was harvested by centrifugation.

Analysis of Process Parameters:

The following process parameters were routinely analysed. The $pO_2$, pH, temperature and stirrer speed were measured online throughout the process time. The optical density was measured offline at 600 nm. The determination of the β-galactosidase activity was performed using a β-Gal Assay Kit (Invitrogen, cat. no. K1455-01). The activity was specified as units per mL $OD_{600}$ = 1.0. It is defined as the quantity of Ortho-Nitrophenyl-β-D-Galactopyranosid (ONPG) in nmol, which is hydrolysed per minute and mL bacteria suspension ($OD_{600}$ = 1.0). The accumulated product was analysed by SDS-PAGE, the total protein content (soluble and insoluble protein) was determined and using HPLC analysis, the soluble fraction was measured. Cell disruption of E.coli was performed in lysis buffer (50 mM glucose, 25 mM tris/HCl (pH 8), 15 mM EDTA (pH 8.0)) with and ultrasonic homogeniser (Bandlin Sonoplus, HD2070). 250 μL bacteria suspension with an $OD_{600}$ of 50 were centrifuged with 14000 RPM for 10 min. The pellet was resuspended in 250 μL lysis buffer (vortex) and placed at room temperature for 5 min. Afterwards, the cells were disrupted for 20 s with ultrasonic at 10 % device performance (cells on ice) and then the cell suspension was centrifuged at 14000 RPM, 10 min. The supernatant (soluble protein) was then analysed by SDS-PAGE and HPLC.

Samples before induction and at end of production (after 5h induction) were taken from the bioreactor for analysis of Qβ formation analyzed by SDS-PAGE standardized to OD 5.0. At the end of cultivation, 1.9 1 of the culture was harvested. After centrifugation, the following cell pellets were obtained in three independent reactor runs: 1.) End $OD_{600}$ of 84: 194 g CWW; 2.) End $OD_{600}$ of 88: 200 g CWW; 3.) End $OD_{600}$ of 86: 201 g CWW.

The plasmid retention in run 1 and 2 was 100 % at induction start and 100 % at harvest. Based on comparison with a Qβ CP standard on SDS-PAGE the yield was roughly estimated to be about 5 g/l Qβ CP. HPLC analysis revealed a concentration of about 6 g/l Qβ VLP.

Example 7

**Selection of carbon source and bacterial strain**

Glucose and glycerol as carbon sources were compared. In order to test the growth behaviour of each of the strains DH20 and RB791 on these carbon sources, shake flask experiments were conducted with medium containing glucose (R27) and medium containing glycerol (R40). Both media were supplemented with 25 μg/ml kanamycin. Each culture was started with an initial $OD_{600}$ of 0.3. Induction was performed by adding 0.5% lactose. The maximum specific growth rates ($\mu_{max}$) and the yield coefficients ($Y_{x/s}$) were determined and are listed in Table 4. RB791 grew faster on both, glucose and glycerol. In addition, the resulting yield coefficients were higher. Although glucose allowed higher maximum specific growth rates ($\mu_{max}$) the yield coefficients ($Y_{x/s}$) was higher for glycerol.

Table 4: Maximum specific growth rates and the yield coefficients of the cultivation experiments with RB791 and DH20 on glucose and glycerol.

| Strain | Carbon source | Value after 4.5 h Culture Time | | Max. spec. growth rate ($\mu_{max}$) [$h^{-1}$] | Yield coefficient ($Y_{x/s}$) biomass from substrate [g/g] |
|---|---|---|---|---|---|
| | | $OD_{600}$ | Acetate [$gl^{-1}$] | | |
| RB791 | glucose | 6.24 | 0.44 | 0.71 | 0.72 |
| | glycerol | 4.04 | 0.21 | 0.62 | 0.86 |
| DH20 | glucose | 2.52 | 0.42 | 0.51 | 0.71 |
| | glycerol | 2.82 | 0.25 | 0.50 | 0.81 |

Example 8

**Determination of Optimal Temperature**

The influence of temperature on product formation was investigated. Two shake flask cultures were inoculated and incubated at 30°C and 220 rpm. After an $OD_{600}$ of 5 was reached, the cultures were induced with lactose. Subsequently, one culture was continued to be incubated at 37°C and the other culture at 23°C. Results of the SDS-PAGE revealed that expression levels at 4 and 5 h after induction are higher in the culture induced at 37°C. Induction of the cultures for 19 h showed a higher Qβ level in the cultures induced at 23°C.

Example 9

**Induction by Co-Feed of lactose and glycerol**

A feed solution of 20 % glycerol and 20% lactose was composed (R42) and applied to fermentation as described in Example 6 at induction start. Figure 1 provides an overview over relevant process parameters throughout the entire process time. Expression was induced at 13.5 h at an $OD_{600}$ of about 55. Upon induction, the feed pump rate was set to constant. Glycerol did not accumulate with feeding. Lactose accumulated to 4 g/l and then it started to diminish. The β-galactosidase activity rose to 10 U/ml and decreased thereafter. Compared with the previous fermentation runs a.) lactose applied as a single lactose pulse at induction start, no feeding; b.) continuous lactose feed without glycerol, the activity was with 7 U/ml higher and the maximum activity was already reached after 2 h as compared to 4 h in runs a.) and b.).

Example 10

**Plasmid retention**

The effect of the following operating conditions on the plasmid retention was tested in the process described in Example 6: 1.) Preculture starting volume, 2.) Kanamycin in the preculture, 3.) Growth and/or induction at 37°C vs. 30°C. The results are summarised in Table 5. Precultures were started with volumes of 5 μl out of the cell bank vial. Inoculation of a small volume allowed growth of a preculture over-night. The preculture for QT0103_F8 contained 25 mg/l kanamycin, whereas the preculture for QT0103_F7 did not contain any kanamycin. Both fermentations were operated at 30°C and induced for 5 h. Judging from the plasmid retentions before and after 5 h induction, supplementing the preculture with kanamycin has a positive effect on plasmid retention. Plasmid retention remained at 98% before and after 5 h induction. In contrast, plasmid retentions reached only values of 80% when kanamycin was omitted from the preculture. For a subsequent run, QT0203_F7, the preculture was also started with 5 μl and grown in kanamycin containing medium. The resulting fermentation in the bioreactor was operated at 37°C from the beginning. Operation at 37 °C had a detrimental effect on the plasmid stability. While the plasmid retention was at 99% before induction, it dropped to 0% after 5 h induction. In order to test whether a shorter preculture and thus, less generations, would improve the plasmid retention after 5 h induction, a set of precultures were started with 300 μl volume from a thawed cell bank vial and grown in kanamycin free medium. Two fermenters were operated at 30°C for the whole run. An additional two fermenters were operated first at 30°C for cell growth and than switched to 37°C for the induction phase. The resulting plasmid stabilities were all at 100% before and 5 h after induction.

Table 5: Summary of plasmid retention before and 5h after induction obtained under different operating conditions in terms of generations in the preculture, with and without kanamycin in the preculture, and growth and/or induction at 37°C.

| Bioreactor run | Preculture Starting Culture Volume [μl] | Kanamycin in preculture | Plasmid retention before induc. [%] | Plasmid retention after 5h induc. [%] | Remarks |
|---|---|---|---|---|---|
| QT0103_F8 | 5 | 25mg/L | 98 | 98 | whole process at 30°C |
| QT0103_F7 | 5 | no | 80 | 80 | whole process at 30°C |
| QT0203_F7 | 5 | 25mg/L | 99 | 0 | Bioreactor run at 37°C |
| QT0603_F7 | 300 | no | 100 | 100 | whole process at 30°C |
| QT0703_F8 | 300 | no | 100 | 100 | Induction at 37°C, rest of the process at 30°C |
| QT0803_F9 | 300 | no | 100 | 100 | whole process at 30°C |
| QT0903_F10 | 300 | no | 100 | 100 | Induction at 37°C, rest of the process at 30°C |

Example 11

**Variation in Time Point of Induction**

In a process essentially as described in Example 6 the exponential feed profile was programmed to start 7 h after the inoculation of the bioreactor. Under standard conditions, the scheduled time for induction was at 14 h process time. In order to test the effect of variations in the time point of induction on the final cell densities, one culture was induced at 13.5 h (resulting in 6.5 h of exponential feed) and another culture at 14.5 h (resulting in 7.5 h of exponential feed). One culture induced at the regular 14 h time point served as a control (7 h of exponential feed). Results are summarised in Table 6. Cell density increased with increasing length of feeding. Judged from a linear regression analysis of the available data points for final CWW, a linear relationship appears to exist ($r^2$ = 0.92).

Table 6: Variations in time point of induction: effect on final cell density in terms of $OD_{600}$ and CWW.

| Reactor | Process Time Point of Induction | Duration of Exp. Feed Phase [h] | Final $OD_{600}$ | Final CWW [g/L] |
|---|---|---|---|---|
| F2 | 13h32min | 6.5 | 83.4 | 116.5 |
| F1 | 14h02min | 7.0 | 82.4 | 122.5 |
| F3 | 14h29min | 7.5 | 100.4 | 141.1 |

Example 12

**Variation in Time point of harvest**

Harvest of the culture in a process essentially as described in Example 6 is performed manually. Under standard conditions, the scheduled time for harvest was at 19 h process time. The operation "Harvest" involves the manual ending of the bioreactor operations. In order to test the effect of variations in the time point of harvest on the final cell densities, one culture was harvested at 18.8 h (resulting in 4.8 h of induction) and another culture at 19.5 h (resulting in 5.5 h of induction). One culture harvested at the regular 19 h time point served as a control (5 h of induction). Results are summarized in Table 7. Cell density increased with increasing length of induction because the cells are still growing while induced.

Table 7: Variation in time point of harvest: effect on fmal cell density in terms of OD600 and CWW.

| Reactor | Process Time Point of Harvest | Length of Induction [h] | Final $OD_{600}$ | Final CWW [g/L] |
|---|---|---|---|---|
| F5 | 18h50min | 4.8 | 91.4 | 122.4 |
| F4 | 19h00min | 5.0 | 92.2 | 127.5 |
| F6 | 19h30min | 5.5 | 96.0 | 132.4 |

Example 13

**Effect of Temperature**

The effect of fermentation temperature in a process essentially as described in Example 6 was investigated by running 6 fermentations at 5 different temperature setpoints. Results are summarized in Table 8. Final cell densities were sensitive to the fermentation temperature with an optimum at a temperature of 30°C.

Table 8: Summarized results of different temperature setpoints on final cell density in terms of OD600 and CWW.

| Reactor | Temperature [°C] | Final $OD_{600}$ | Final CWW [g/L] |
|---|---|---|---|
| F5 | 25.0 | 37.8 | 62 |
| F4 | 27.5 | 80.0 | 117 |
| F3 | 30.0 | 92.8 | 123 |
| F4 | 30.0 | 92.4 | 125 |
| F5 | 32.5 | 85.0 | 111 |
| F6 | 35.0 | 79.6 | 107 |

Example 14

**Up-Scaled Fermentation at 50 l Scale**

The process described in Example 6 was scaled up to a volume of 50 1 order to evaluate upscale capability from the 2 L working volume bioreactor system to a larger volume. Key process parameters for the upscaled process are summarized in Table 9.

Table 9: Process parameters of in 50 L bioreactor.

| Culture Step | Description | Time [h] | $OD_{600}$ |
|---|---|---|---|
| Preculture 1 | 300 μl from cell bank vial are transferred into 100 ml preculture medium contained in 500 mL shake flask and cultured for 16 h | -11* | 5.0 |
| Preculture 2 | Calculate the required volume for transfer in order to start with initial $OD_{600}$ of 0.3 in 750 ml. Tranfer calculated volume (e.g. 50 ml) into 750 ml preculture medium contained in 5000 mL shake flask | -5* | 4.0 |
| Inoculation of Bioreactor | Pooled calculated volume (e.g. 1.4 L) is transferred into the 50 L Bioreactor. Initial volume: =40L | 0 | |
| Induction Start | The exponential feeding profile is switched to constant and feed is switched to induction feed | 14 | 46 |
| End of Culture | Culture is completed after 5 h of induction | 19 | 128 |
| * Relative to the time of bioreactor inoculation. | | | |

It was necessary to have two preculture expansion steps. In the first step, the cells were expanded as established for the 2 L process (Example 6). After this step, cells were split into two 5000 ml shake flask cultures, containing 750 ml medium each. Further expansion was performed for 5 h. The cultures in the 50 L bioreactors were performed with the same time profile as in the 2 L system (Example 6). $OD_{600}$ at induction start was 46, the final $OD_{600}$ was 128. Plasmid retention was 100 % before induction and 98 % at the end of culture. The concentration of Qβ CP protein in the medium at the end of culture was roughly estimated 8 g/l using SDS-PAGE. The total amount of Qβ was estimated about 300 g for this reactor run.

Example 15

**Effect of Extended exponential feed**

The exponential feeding phase for fermentations performed according to Examples 6 or 14 was 7 h. After this time the cells reached a density for induction, which increased during induction to the targeted maximum $OD_{600}$ of around 100 to 130 as final cell density. Final $OD_{600}$, final CWW, final CDW, plasmid retention at induction start and harvest and Qβ concentration at the end of culture are determined for reactor runs performed as described in Examples 6 and 14, , preferably as in Example 14, wherein the exponential feeding phase is extended to a duration up to 11 h, preferably to 10 h.

Example 16

**Effect of increased feeding during production**

Example 9 demonstrates that the glycerol does not accumulate during production phase, indicating that production might be limited by the feeding rate of induction medium. Effect of extended feeding rate of induction medium on final $OD_{600}$, final CWW, final CDW, plasmid retention at induction start and harvest and Qβ concentration at the end of culture is determined in reactor runs as described in Example 6 and 14, preferably as in Example 14, wherein the feeding rate during production is increased. Alternatively or additionally, the ratio between lactose and glycerol in the feed medium shifted towards a higher glycerol and a lower lactose concentration.

Example 17

**HPLC Analysis of Qβ CP**

Qβ CP was measured with an HPLC system as follows: A sample containing Qβ CP was diluted appropriately in 1x reaction buffer (50 mM tris(hydroxymethyl)aminomethane buffer pH 8.0) containing 10 mM 1,4-Dithio-DL-threitol and incubated for 15 min at 50°C in a thermomixer. After incubation the sample was centrifuged and the supernatant was stored at 2°C to 10°C until HPLC analysis. 10 to 100 μl of the sample are injected.

Qβ was quantified with a regression curve of known Qβ standards regressed to the HPLC peak area detected at 215 nm after elution from a $C_4$ reversed phase column, 300 Å, 5μm, 4.6 x 150 mm, Vydac Inc., Hesperia, USA (Cat. No. 214TP5415) thermally equilibrated at 50°C. The flow rate through the system was 1 ml/min consisting of mobile phase A (0.12 % trifluoroacetic acid in water) and mobile phase B (0.12 % trifluoroacetic acid in acetonitrile) with the following gradient of phase B: 0 to 2 min constant at 40%, 2 to 8 min linear increase to 50%, 8 to 10 min constant at 50%, 10 to 10.1 min linear decrease to 40%, and 10.1 to 12 min constant at 40%.

Example 18

**Determination of Qβ VLP by analytical size exclusion chromatography**

Analysis of Qβ particles by analytical size exclusion chromatography is performed using a TskgelG5000 $PW_{XL}$-column (10 μm, 7.8 x 300 mm, TosoH Biosep; Cat.—No. 08023) equilibrated in phosphate buffered saline (20 mM $Na_2HPO_4$/$N_aH_2PO_4$, 150 mM NaCl pH 7.2). Elution is performed by an isocratic gradient for 20 min at 0.8 ml/min in phosphate buffered saline. The Qbeta concentration is determined from a regression curve of known Qβ standards regressed to the HPLC peak area detected at 260 nm.

## Claims

**1.** A process for expression of a recombinant capsid protein of a bacteriophage or a mutant or fragment thereof being capable of forming a VLP by self-assembly, said process comprising the steps of:

a.) introducing an expression plasmid into a bacterial host, wherein said expression plasmid comprises a nucleotide sequence encoding said recombinant capsid protein, or mutant or fragment thereof, and a promoter being inducible by lactose, and wherein said bacterial host overexpresses the repressor lacI;
b.) introducing said bacterial host into a batch culture;
c.) cultivating said bacterial host in a medium comprising a major carbon source and under conditions under which said promoter is repressed by lacI;
d.) feeding said batch culture with said major carbon source; and
e.) inducing said promoter with an inducer, wherein said feeding of said batch culture with said major carbon source is continued.

**2.** The process of claim 1, wherein said bacteriophage is a RNA phage.

**3.** The process of claim 1 or 2, wherein said bacteriophage is selected from the group consisting of:

a.) bacteriophage Qβ;
b.) bacteriophage AP205;
c.) bacteriophage fr;
d.) bacteriophage GA;
e.) bacteriophage SP;
f.) bacteriophage MS2;
g.) bacteriophage M11;
h.) bacteriophage MX1;
i.) bacteriophage NL95;
j.) bacteriophage f2;
k.) bacteriophage PP7 and
l.) bacteriophage R17.

**4.** The process of claim 2, wherein said RNA phage is Qβ.

**5.** The process of claim 4, wherein said recombinant capsid protein has the amino acid sequence of SEQ ID NO:5.

**6.** The process of any one of claims 1 to 5, wherein said expression plasmid comprises a first stop codon and a second stop codon, wherein said first stop codon is located directly 3' of said nucleotide sequence and wherein said second stop codon is located directly 3' of said first stop codon, and wherein at least one of said first or second stop codon is TAA.

**7.** The process of claim 1, wherein said expression plasmid comprising said nucleotide sequence comprises an additional nucleotide sequence, wherein said nucleotide sequence is encoding Qβ CP, or a mutant or fragment thereof, and wherein said additional nucleotide sequence is encoding the Qβ A1 protein or a mutant or fragment thereof, and wherein said nucleotide sequence and said additional nucleotide sequence are separated by exactly one sequence stretch comprising at least one TAA stop codon.

**8.** The process of claim 1, wherein said expression plasmid comprises the nucleotide sequence of SEQ ID NO:6.

**9.** The process of claim 1, wherein said expression plasmid has the nucleotide sequence of SEQ ID NO:1.

**10.** The process of claim 4, wherein said recombinant capsid protein is mutated.

**11.** The process of any one of claims 1 to 7, wherein said promoter is selected from the group consisting of the

a.) tac promoter;
b.) trc promoter;
c.) tic promoter;
d.) lac promoter;
e.) lacUV5 promoter;
f.) $P_{syn}$ promoter;
g.) $lpp^{a}$ promoter;
h.) lpp-lac promoter;
i.) T7-lac promoter;
j.) T3-lac promoter;
k.) T5-lac promoter; and
l.) a promoter having at least 50 % sequence homology to SEQ ID NO:2.

**12.** The process of any one of claims 1 to 11, wherein said promoter comprises the nucleotide sequence of SEQ ID NO:2.

**13.** The process of any one of claims 1 to 12, wherein said major carbon source is glycerol.

**14.** The process of any one of claims 1 to 13, wherein said feeding of said batch culture in step d.) of claim 1 is performed with a flow rate, wherein said flow rate increases with an exponential coefficient $\mu$, and wherein preferably said exponential coefficient $\mu$ is below $\mu_{max}$.

**15.** The process of any one of claims 1 to 14, wherein said inducing of said promoter in step e.) of claim 1 is performed by co-feeding said batch culture with said inducer and said major carbon source at a constant flow rate.

**16.** The process of any one of claims 1 to 14, wherein said inducing of said promoter in step e.) of claim 1 is performed by co-feeding said batch culture with said inducer and said major carbon source at an increasing flow rate.

**17.** The process of claim 15 or claim 16, wherein said inducer is lactose and wherein preferably said lactose and said major carbon source are co-fed to said batch culture in a ratio of about 2:1 to 1:3 (w/w).

**18.** The process of any one of claims 1 to 16, wherein said inducer is IPTG and wherein preferably the concentration of said IPTG in said batch culture is 0.001 to 5 mM.

**19.** The process of claim 18, wherein the concentration of said IPTG in said batch culture is about 0.01 mM.

**20.** The process of any one of claims 1 to 19, wherein said bacterial host comprises the $lacI^{q}$ gene on its chromosome.

**21.** The process of any one of claims 1 to 20, wherein said bacterial host comprises the $lacI^{q}$ gene on a plasmid.

**22.** The process of any one of claims 1 to 21, wherein said cultivating and said feeding of said batch culture and said inducing of said promoter is performed at a temperature which is below the optimal growth temperature of said bacterial host.

**23.** The process of claim 9 wherein:

a.) said major carbon source is glycerol;

b.) said feeding of said batch culture is performed with a flow rate, wherein said flow rate increases with an exponential coefficient $\mu$, and wherein preferably said exponential coefficient $\mu$ is below $\mu_{max}$;
c.) said inducer is lactose;
d.) and said lactose and said major carbon source are co-fed to said batch culture in a ratio of about 2:1 to 1:3 (w/w), preferably 1:1 (w/w), most preferably 2:1 (w/w);
e.) said bacterial host is E. coli RB791; and
f.) said cultivating and feeding of said batch culture and said inducing of said promoter is performed at a temperature of about 30 °C.

**24.** The process of claim 1 wherein:

a.) said expression plasmid has the nucleotide sequence of SEQ ID NO:30;
b.) said the major carbon source is glycerol;
c.) said feeding of said batch culture is performed with a flow rate, wherein said flow rate increases with an exponential coefficient $\mu$, and wherein preferably said exponential coefficient $\mu$ is below $\mu_{max}$;
d.) said inducer is lactose;
e.) said lactose and said major carbon source are co-fed to the batch culture in a ratio of about 2:1 to 1:3 (w/w), preferably 1:1 (w/w), most preferably 2:1 (w/w);
f.) said bacterial host is E. coli RB791; and
g.) said cultivating and feeding of said batch culture and said inducing of said promoter is performed at a temperature of about 30 °C.

**Figure 1**

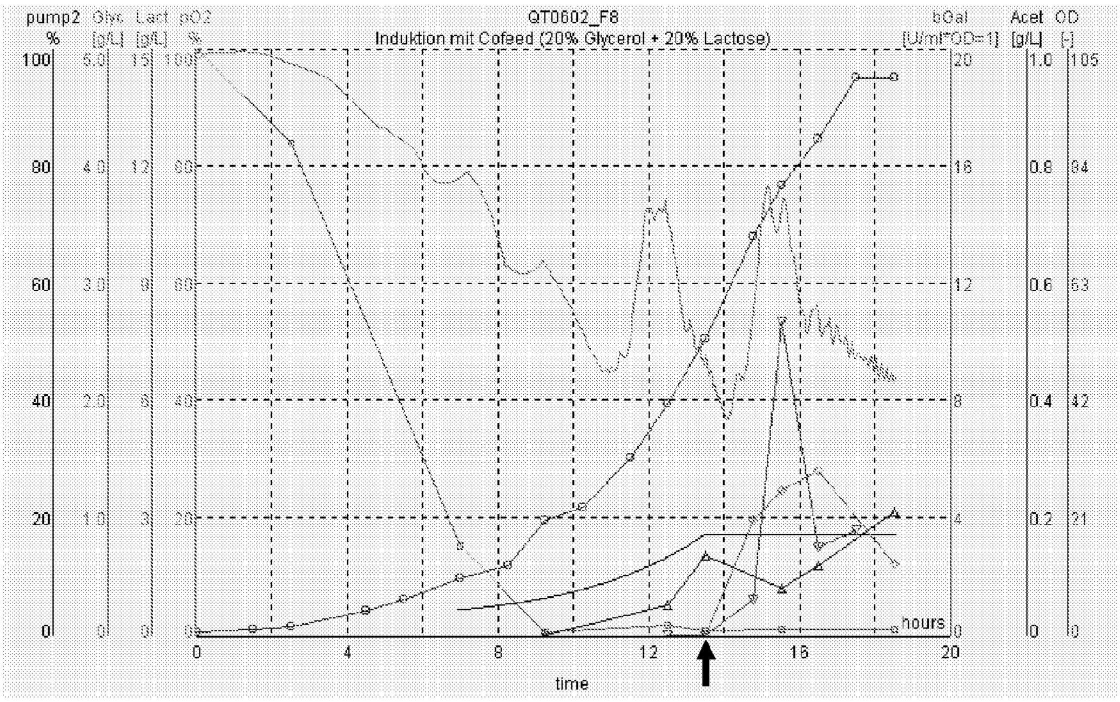
pump2 %
Glyc [g/L]
Lact [g/L]
pO2 %
QT0602_F8
Induktion mit Cofeed (20% Glycerol + 20% Lactose)
bGal [U/ml*OD=1]
Acet [g/L]
OD [-]
hours
time

European Patent Office

# EUROPEAN SEARCH REPORT

Application Number
EP 05 10 6729

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 92/07077 A (PERHAM, RICHARD, NELSON; WILLIS, ANNE, ELIZABETH)<br>30 April 1992 (1992-04-30)<br>* abstract *<br>* example 1.2 *<br>* page 8, line 23 - line 26 * | 1,2, 11-22<br><br><br>3-10 | C12P21/02<br>C07K14/08<br>C12N15/72 |
| Y | KOZLOVSKA T M ET AL: "RNA phage Q beta coat protein as a carrier for foreign epitopes"<br>INTERVIROLOGY,<br>vol. 39, no. 1/2, January 1996 (1996-01), pages 9-15, XP002104008<br>ISSN: 0300-5526<br>* the whole document * | 3-10 | |
| A | DONOVAN R S ET AL: "REVIEW: OPTIMIZING INDUCER AND CULTURE CONDITIONS FOR EXPRESSION OF FOREIGN PROTEINS UNDER THE CONTROL OF THE LAC PROMOTER"<br>JOURNAL OF INDUSTRIAL MICROBIOLOGY, SOCIETY FOR INDUSTRILA MICROBIOLOGY, NL,<br>vol. 16, no. 3, 1996, pages 145-154, XP008020707<br>ISSN: 0169-4146<br>* the whole document * | 1-24 | TECHNICAL FIELDS SEARCHED (IPC)<br>C12P<br>C07K<br>C12N |
| A | DONOVAN ROBERT S ET AL: "Optimizing the expression of a monoclonal antibody fragment under the transcriptional control of the Escherichia coli lac promoter"<br>CANADIAN JOURNAL OF MICROBIOLOGY,<br>vol. 46, no. 6, June 2000 (2000-06), pages 532-541, XP008057798<br>ISSN: 0008-4166<br>* abstract *<br>-/-- | 1-24 | |

The present search report has been drawn up for all claims

| Place of search | Date of completion of the search | Examiner |
|---|---|---|
| The Hague | 3 January 2006 | Huse, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

European Patent Office

# EUROPEAN SEARCH REPORT

Application Number
EP 05 10 6729

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | WO 92/13081 A (BRITISH TECHNOLOGY GROUP PLC) 6 August 1992 (1992-08-06)<br>* abstract *<br>* examples 1a),c) *<br>----- | 1-24 | |
| D,A | PUSHKO P ET AL: "Analysis of RNA phage fr coat protein assembly by insertion, deletion and substitution mutagenesis"<br>PROTEIN ENGINEERING, OXFORD UNIVERSITY PRESS, SURREY, GB,<br>vol. 6, no. 8, 1993, pages 883-891, XP002104010<br>ISSN: 0269-2139<br>* abstract *<br>* page 884, left-hand column, paragraph 1 *<br>----- | 1-24 | |
| | | | TECHNICAL FIELDS SEARCHED (IPC) |

The present search report has been drawn up for all claims

| Place of search | Date of completion of the search | Examiner |
|---|---|---|
| The Hague | 3 January 2006 | Huse, I |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 10 6729

This annex lists the patent family members relating to the patent documents cited in the above-mentioned European search report.
The members are as contained in the European Patent Office EDP file on
The European Patent Office is in no way liable for these particulars which are merely given for the purpose of information.

03-01-2006

| Patent document cited in search report | Publication date | Patent family member(s) | Publication date |
|---|---|---|---|
| WO 9207077 A | 30-04-1992 | AT 149202 T | 15-03-1997 |
| | | DE 69124800 D1 | 03-04-1997 |
| | | DE 69124800 T2 | 31-07-1997 |
| | | DK 552267 T3 | 21-07-1997 |
| | | EP 0552267 A1 | 28-07-1993 |
| | | ES 2100240 T3 | 16-06-1997 |
| | | GR 3023393 T3 | 29-08-1997 |
| | | JP 6501848 T | 03-03-1994 |
| WO 9213081 A | 06-08-1992 | AT 201449 T | 15-06-2001 |
| | | AU 653387 B2 | 29-09-1994 |
| | | AU 1173792 A | 27-08-1992 |
| | | CA 2101070 A1 | 25-07-1992 |
| | | DE 69231837 D1 | 28-06-2001 |
| | | DE 69231837 T2 | 18-10-2001 |
| | | EP 0572433 A1 | 08-12-1993 |
| | | FI 933328 A | 07-09-1993 |
| | | GB 2253626 A | 16-09-1992 |
| | | IE 920200 A1 | 29-07-1992 |
| | | JP 3187832 B2 | 16-07-2001 |
| | | JP 6504907 T | 09-06-1994 |
| | | KR 197819 B1 | 15-06-1999 |
| | | NO 932644 A | 23-09-1993 |
| | | NZ 241373 A | 28-04-1993 |
| | | US 5534257 A | 09-07-1996 |
| | | ZA 9200511 A | 25-11-1992 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02056905 A
- WO 9213081 A
- WO 2004007538 A


### Non-patent literature cited in the description


- **KASTELEIN et al.** *Gene,* 1983, vol. 23, 245-254
- **KOZLOVSKAYA et al.** *Dokl. Akad. Nauk SSSR,* 1986, vol. 287, 452-455
- **PUSHKO et al.** *Protein Engineering,* 1993, vol. 6 (8), 883-891
- **KOZLOVSKA et al.** *Gene,* 1993, vol. 137, 133-137
- **CILIENS et al.** *FEBS Letters,* 2000, vol. 24171, 1-4
- **VASILJEVA et al.** *FEBS Letters,* 1998, vol. 431, 7-11
- **MASTICO et al.** *Journal of General Virology,* 1993, vol. 74, 541-548
- **HEAL et al.** *Vaccine,* 2000, vol. 18, 251-258
- **PEABODY ; AL-BITAR.** *Nucleic Acid Research,* 2001, vol. 29 (22), e113
- **STOLL, E. et al.** *J. Biol. Chem.,* 1977, vol. 252, 990-993
- **GOLMOHAMMADI, R. et al.** *Structure,* 1996, vol. 4, 543-5554
- **KOZLOVSKA et al.** *Intervirology,* 1996, vol. 39, 9-15
- **DONAVAN et al.** *Can. J. Microbiol,* 2000, vol. 46, 532-541
- **MAKRIDES.** *Microbiological Reviews,* 1996, 512-538
- **MAKRIDES.** *Microbiol. Rev.,* 1996, vol. 60, 512-538
- **GOLDSTEIN ; DOI.** *Biotechnology Annual Review,* 1995, vol. 1, 105-128
- **HANNIG ; MAKRIDES.** *TIBTECH,* 1998, vol. 16, 54-60
- **STEVENS.** *Structures,* 2000, vol. 8, R177-R185
- **DE BOER et al.** *PNAS,* 1983, vol. 80, 21-25
- **AMANN et al.** *Gene,* 1983, vol. 25, 167-178
- **BROSIUS et al.** *Journal of Biological Chemistry,* 1985, vol. 260 (6), 3539-3541
- **DALBØGE et al.** *DNA,* 1988, vol. 7 (6), 399-405
- **RINGQUIST et al.** *Mol. Micr.,* 1992, vol. 6, 1219-1229
- **MENZELLA et al.** *Biotechnology and Bioengineering,* 2003, vol. 82 (7), 809-817
- **GLASCOCK ; WEICKERT.** *Gene,* 1998, vol. 223 (1-2), 221-231
- **KOPETZKI et al.** *Mol Gen Genet,* 1989, vol. 216, 149-155

SEQUENCE LISTING

<110> Cytos Biotechnology AG

<120> SCALABLE FERMENTATION PROCESS

<130> P1049EP01

<160> 40

<170> PatentIn version 3.3

<210> 1
<211> 5579
<212> DNA
<213> artificial sequence

<220>
<223> plasmid

<400> 1
ggctgtgcag gtcgtaaatc actgcataat tcgtgtcgct caaggcgcac tcccgttctg 60

gataatgttt tttgcgccga catcataacg gttctggcaa atattctgaa atgagctgtt 120

gacaattaat catcggctcg tataatgtgt ggaattgtga gcggataaca atttcacaca 180

ggaaacagaa ttctaaggag gaaaaaaaaa tggcaaaatt agagactgtt actttaggta 240

acatcgggaa agatggaaaa caaactctgg tcctcaatcc gcgtggggta aatcccacta 300

acggcgttgc ctcgctttca caagcgggtg cagttcctgc gctggagaag cgtgttaccg 360

tttcggtatc tcagccttct cgcaatcgta agaactacaa ggtccaggtt aagatccaga 420

acccgaccgc ttgcactgca aacggttctt gtgacccatc cgttactcgc caggcatatg 480

ctgacgtgac cttttcgttc acgcagtata gtaccgatga ggaacgagct tttgttcgta 540

cagagcttgc tgctctgctc gctagtcctc tgctgatcga tgctattgat cagctgaacc 600

cagcgtatta atgactgctc attgccggtg gtggctcagg gtcaaaaccc gatccggtta 660

ttccggatcc accgattgat ccgccgccag ggacaggtaa gtatacctgt cccttcgcaa 720

tttggtccct agaggaggtt tacgagcctc ctactaagaa ccgaccgtgg cctatctata 780

atgctgttga actccagcct cgcgaatttg atgttgccct caaagatctt ttgggcaata 840

caaagtggcg tgattgggat tctcggctta gttataccac gttccgcggt tgccgtggca 900

atggttatat tgaccttgat gcgacttatc ttgctactga tcaggctatg cgtgatcaga 960

agtatgatat tcgcgagggc aagaaacctg gtgctttcgg taacattgag cgattcattt 1020

atcttaagtc gataaatgct tattgctctc ttagcgatat tgcggcctat cacgccgatg 1080

gcgtgatagt tggcttttgg cgcgatccat ccagtggtgg tgccataccg tttgacttca 1140

ctaagtttga taagactaaa tgtcctattc aagccgtgat agtcgttcct cgtgcttagt 1200

aactaaggat gaaatgcatg tctaagcttg gctgttttgg cggatgagag aagattttca 1260

gcctgataca gattaaatca gaacgcagaa gcggtctgat aaaacagaat ttgcctggcg 1320
gcagtagcgc ggtggtccca cctgacccca tgccgaactc agaagtgaaa cgccgtagcg 1380
ccgatggtag tgtggggtct ccccatgcga gagtagggaa ctgccaggca tcaaataaaa 1440
cgaaaggctc agtcgaaaga ctgggccttt cgttttatct gttgtttgtc ggtgaacgct 1500
ctcctgagta ggacaaatcc gccgggagcg gatttgaacg ttgcgaagca acggcccgga 1560
gggtggcggg caggacgccc gccataaact gccaggcatc aaattaagca gaaggccatc 1620
ctgacggatg gcctttttgc gtttctacaa actcttttgt ttatttttct agagccacgt 1680
tgtgtctcaa aatctctgat gttacattgc acaagataaa aatatatcat catgaacaat 1740
aaaactgtct gcttacataa acagtaatac aaggagtgtt atgagccata ttcaacggga 1800
aacgtcttgc tcgaggccgc gattaaattc caacatggat gctgatttat atgggtataa 1860
atgggctcgc gataatgtcg ggcaatcagg tgcgacaatc tatcgattgt atgggaagcc 1920
cgatgcgcca gagttgtttc tgaaacatgg caaaggtagc gttgccaatg atgttacaga 1980
tgagatggtc agactaaact ggctgacgga atttatgcct cttccgacca tcaagcattt 2040
tatccgtact cctgatgatg catggttact caccactgcg atccccggga aaacagcatt 2100
ccaggtatta gaagaatatc ctgattcagg tgaaaatatt gttgatgcgc tggcagtgtt 2160
cctgcgccgg ttgcattcga ttcctgtttg taattgtcct tttaacagcg atcgcgtatt 2220
tcgtctcgct caggcgcaat cacgaatgaa taacggtttg gttgatgcga gtgattttga 2280
tgacgagcgt aatggctggc ctgttgaaca agtctggaaa gaaatgcata agcttttgcc 2340
attctcaccg gattcagtcg tcactcatgg tgatttctca cttgataacc ttatttttga 2400
cgaggggaaa ttaataggtt gtattgatgt tggacgagtc ggaatcgcag accgatacca 2460
ggatcttgcc atcctatgga actgcctcgg tgagttttct ccttcattac agaaacggct 2520
ttttcaaaaa tatggtattg ataatcctga tatgaataaa ttgcagtttc atttgatgct 2580
cgatgagttt ttctaaacgc gtgaccaagt ttactcatat gtactttaga ttgatttaaa 2640
acttcatttt taatttaaaa ggatctaggt gaagatcctt tttgataatc tcatgaccaa 2700
aatcccttaa cgtgagtttt cgttccactg agcgtcagac cccgtagaaa agatcaaagg 2760
atcttcttga gatccttttt ttctgcgcgt aatctgctgc ttgcaaacaa aaaaaccacc 2820
gctaccagcg gtggtttgtt tgccggatca agagctacca actctttttc cgaaggtaac 2880
tggcttcagc agagcgcaga taccaaatac tgtccttcta gtgtagccgt agttaggcca 2940
ccacttcaag aactctgtag caccgcctac atacctcgct ctgctaatcc tgttaccagt 3000
ggctgctgcc agtggcgata agtcgtgtct taccgggttg gactcaagac gatagttacc 3060
ggataaggcg cagcggtcgg gctgaacggg gggttcgtgc acacagccca gcttggagcg 3120

```
aacgacctac accgaactga gatacctaca gcgtgagcta tgagaaagcg ccacgcttcc   3180
cgaagggaga aaggcggaca ggtatccggt aagcggcagg gtcggaacag gagagcgcac   3240
gagggagctc ccagggggaa acgcctggta tctttatagt cctgtcgggt ttcgccacct   3300
ctgacttgag cgtcgatttt tgtgatgctc gtcagggggg cggagcctat ggaaaaacgc   3360
cagcaacgcg gcctttttac ggttcctggc cttttgctgg ccttttgctc acatgttctt   3420
tcctgcgtta tcccctgatt ctgtggataa ccgtattacc gcctttgagt gagctgatac   3480
cgctcgccgc agccgaacga ccgagcgcag cgagtcagtg agcgaggaag cggaagagcg   3540
cctgatgcgg tattttctcc ttacgcatct gtgcggtatt tcacaccgca tatggtgcac   3600
tctcagtaca atctgctctg atgccgcata gttaagccag tatacactcc gctatcgcta   3660
cgtgactggg tcatggctgc gccccgacac ccgccaacac ccgctgacgc gccctgacgg   3720
gcttgtctgc tcccggcatc cgcttacaga caagctgtga ccgtctccgg gagctgcatg   3780
tgtcagaggt tttcaccgtc atcaccgaaa cgcgcgaggc agctgcggta aagctcatca   3840
gcgtggtcgt gaagcgattc acagatgtct gcctgttcat ccgcgtccag ctcgttgagt   3900
ttctccagaa gcgttaatgt ctggcttctg ataaagcggg ccatgttaag ggcggttttt   3960
tcctgtttgg tcactgatgc ctccgtgtaa gggggatttc tgttcatggg ggtaatgata   4020
ccgatgaaac gagagaggat gctcacgata cgggttactg atgatgaaca tgcccggtta   4080
ctggaacgtt gtgagggtaa acaactggcg gtatggatgc ggcgggacca gagaaaaatc   4140
actcagggtc aatgccagcg cttcgttaat acagatgtag gtgttccaca gggtagccag   4200
cagcatcctg cgatgcagat ccggaacata atggtgcagg gcgctgactt ccgcgtttcc   4260
agactttacg aaacacggaa accgaagacc attcatgttg ttgctcaggt cgcagacgtt   4320
ttgcagcagc agtcgcttca cgttcgctcg cgtatcggtg attcattctg ctaaccagta   4380
aggcaacccc gccagcctag ccgggtcctc aacgacagga gcacgatcat gcgcacccgt   4440
ggccaggacc caacgctgcc cgagatgcgc cgcgtgcggc tgctggagat ggcggacgcg   4500
atggatatgt tctgccaagg gttggtttgc gcattcacag ttctccgcaa gaattgattg   4560
gctccaattc ttggagtggt gaatccgtta gcgaggtgcc gccggcttcc attcaggtcg   4620
aggtggcccg gctccatgca ccgcgacgca acgcggggag gcagacaagg tatagggcgg   4680
cgcctacaat ccatgccaac ccgttccatg tgctcgccga ggcggcataa atcgccgtga   4740
cgatcagcgg tccaatgatc gaagttaggc tggtaagagc cgcgagcgat ccttgaagct   4800
gtccctgatg gtcgtcatct acctgcctgg acagcatggc ctgcaacgcg ggcatcccga   4860
tgccgccgga agcgagaaga atcataatgg ggaaggccat ccagcctcgc gtcgcgaacg   4920
ccagcaagac gtagcccagc gcgtcggccg ccatgccggc gataatggcc tgcttctcgc   4980
```

cgaaacgttt ggtggcggga ccagtgacga aggcttgagc gagggcgtgc aagattccga 5040

ataccgcaag cgacaggccg atcatcgtcg cgctccagcg aaagcggtcc tcgccgaaaa 5100

tgacccagag cgctgccggc acctgtccta cgagttgcat gataaagaag acagtcataa 5160

gtgcggcgac gatagtcatg ccccgcgccc accggaagga gctgactggg ttgaaggctc 5220

tcaagggcat cggtcgacgc tctcccttat gcgactcctg cattaggaag cagcccagta 5280

gtaggttgag gccgttgagc accgccgccg caaggaatgg tgcatgcaag gagatggcgc 5340

ccaacagtcc cccggccacg gggcctgcca ccatacccac gccgaaacaa gcgctcatga 5400

gcccgaagtg gcgagcccga tcttccccat cggtgatgtc ggcgatatag gcgccagcaa 5460

ccgcacctgt ggcgccggtg atgccggcca cgatgcgtcc ggcgtagagg atccgggctt 5520

atcgactgca cggtgcacca atgcttctgg cgtcaggcag ccatcggaag ctgtggtat 5579

<210> 2
<211> 245
<212> DNA
<213> artificial sequence

<220>
<223> promoter sequence

<400> 2
cgactgcacg gtgcaccaat gcttctggcg tcaggcagcc atcggaagct gtggtatggc 60

tgtgcaggtc gtaaatcact gcataattcg tgtcgctcaa ggcgcactcc cgttctggat 120

aatgtttttt gcgccgacat cataacggtt ctggcaaata ttctgaaatg agctgttgac 180

aattaatcat cggctcgtat aatgtgtgga attgtgagcg gataacaatt tcacacagga 240

aacag 245

<210> 3
<211> 19
<212> DNA
<213> artificial sequence

<220>
<223> Shine-Dalgarno Sequence

<400> 3
taaggaggaa aaaaaaatg 19

<210> 4
<211> 18
<212> DNA
<213> artificial sequence

<220>
<223> Shine-Dalgarno Sequence

<400> 4
aggaggtaaa aaacgatg 18

<210> 5
<211> 133
<212> PRT
<213> Bacteriophage Qbeta

<400> 5

Met Ala Lys Leu Glu Thr Val Thr Leu Gly Asn Ile Gly Lys Asp Gly
1 5 10 15

Lys Gln Thr Leu Val Leu Asn Pro Arg Gly Val Asn Pro Thr Asn Gly
20 25 30

Val Ala Ser Leu Ser Gln Ala Gly Ala Val Pro Ala Leu Glu Lys Arg
35 40 45

Val Thr Val Ser Val Ser Gln Pro Ser Arg Asn Arg Lys Asn Tyr Lys
50 55 60

Val Gln Val Lys Ile Gln Asn Pro Thr Ala Cys Thr Ala Asn Gly Ser
65 70 75 80

Cys Asp Pro Ser Val Thr Arg Gln Ala Tyr Ala Asp Val Thr Phe Ser
85 90 95

Phe Thr Gln Tyr Ser Thr Asp Glu Glu Arg Ala Phe Val Arg Thr Glu
100 105 110

Leu Ala Ala Leu Leu Ala Ser Pro Leu Leu Ile Asp Ala Ile Asp Gln
115 120 125

Leu Asn Pro Ala Tyr
130

<210> 6
<211> 1017
<212> DNA
<213> artificial sequence

<220>
<223> Expression construct

<400> 6
atggcaaaat tagagactgt tactttaggt aacatcggga aagatggaaa acaaactctg 60

gtcctcaatc cgcgtggggt aaatcccact aacggcgttg cctcgctttc acaagcgggt 120

gcagttcctg cgctggagaa gcgtgttacc gtttcggtat ctcagccttc tcgcaatcgt 180

aagaactaca aggtccaggt taagatccag aacccgaccg cttgcactgc aaacggttct 240

tgtgacccat ccgttactcg ccaggcatat gctgacgtga ccttttcgtt cacgcagtat 300

agtaccgatg aggaacgagc ttttgttcgt acagagcttg ctgctctgct cgctagtcct 360

ctgctgatcg atgctattga tcagctgaac ccagcgtatt aatgactgct cattgccggt 420

ggtggctcag ggtcaaaacc cgatccggtt attccggatc caccgattga tccgccgcca 480

gggacaggta agtatacctg tcccttcgca atttggtccc tagaggaggt ttacgagcct 540

cctactaaga accgaccgtg gcctatctat aatgctgttg aactccagcc tcgcgaattt 600

gatgttgccc tcaaagatct tttgggcaat acaaagtggc gtgattggga ttctcggctt 660

agttatacca cgttccgcgg ttgccgtggc aatggttata ttgaccttga tgcgacttat 720

cttgctactg atcaggctat gcgtgatcag aagtatgata ttcgcgaggg caagaaacct 780

ggtgctttcg gtaacattga gcgattcatt tatcttaagt cgataaatgc ttattgctct 840

cttagcgata ttgcggccta tcacgccgat ggcgtgatag ttggcttttg gcgcgatcca 900

tccagtggtg gtgccatacc gtttgacttc actaagtttg ataagactaa atgtcctatt 960

caagccgtga tagtcgttcc tcgtgcttag taactaagga tgaaatgcat gtctaag 1017

<210> 7
<211> 132
<212> PRT
<213> Bacteriophage Qbeta

<400> 7

Ala Lys Leu Glu Thr Val Thr Leu Gly Asn Ile Gly Arg Asp Gly Lys
1 5 10 15

Gln Thr Leu Val Leu Asn Pro Arg Gly Val Asn Pro Thr Asn Gly Val
20 25 30

Ala Ser Leu Ser Gln Ala Gly Ala Val Pro Ala Leu Glu Lys Arg Val
35 40 45

Thr Val Ser Val Ser Gln Pro Ser Arg Asn Arg Lys Asn Tyr Lys Val
50 55 60

Gln Val Lys Ile Gln Asn Pro Thr Ala Cys Thr Ala Asn Gly Ser Cys
65 70 75 80

Asp Pro Ser Val Thr Arg Gln Lys Tyr Ala Asp Val Thr Phe Ser Phe
85 90 95

Thr Gln Tyr Ser Thr Asp Glu Glu Arg Ala Phe Val Arg Thr Glu Leu
100 105 110

```
Ala Ala Leu Leu Ala Ser Pro Leu Leu Ile Asp Ala Ile Asp Gln Leu
        115                 120                 125

Asn Pro Ala Tyr
    130


<210>  8
<211>  132
<212>  PRT
<213>  Bacteriophage Qbeta

<400>  8

Ala Lys Leu Glu Thr Val Thr Leu Gly Lys Ile Gly Lys Asp Gly Lys
1               5                   10                  15

Gln Thr Leu Val Leu Asn Pro Arg Gly Val Asn Pro Thr Asn Gly Val
            20                  25                  30

Ala Ser Leu Ser Gln Ala Gly Ala Val Pro Ala Leu Glu Lys Arg Val
        35                  40                  45

Thr Val Ser Val Ser Gln Pro Ser Arg Asn Arg Lys Asn Tyr Lys Val
    50                  55                  60

Gln Val Lys Ile Gln Asn Pro Thr Ala Cys Thr Ala Asn Gly Ser Cys
65                  70                  75                  80

Asp Pro Ser Val Thr Arg Gln Lys Tyr Ala Asp Val Thr Phe Ser Phe
                85                  90                  95

Thr Gln Tyr Ser Thr Asp Glu Glu Arg Ala Phe Val Arg Thr Glu Leu
            100                 105                 110

Ala Ala Leu Leu Ala Ser Pro Leu Leu Ile Asp Ala Ile Asp Gln Leu
        115                 120                 125

Asn Pro Ala Tyr
    130


<210>  9
<211>  132
<212>  PRT
<213>  Bacteriophage Qb

<400>  9

Ala Arg Leu Glu Thr Val Thr Leu Gly Asn Ile Gly Arg Asp Gly Lys
1               5                   10                  15
```

Gln Thr Leu Val Leu Asn Pro Arg Gly Val Asn Pro Thr Asn Gly Val
20 25 30

Ala Ser Leu Ser Gln Ala Gly Ala Val Pro Ala Leu Glu Lys Arg Val
35 40 45

Thr Val Ser Val Ser Gln Pro Ser Arg Asn Arg Lys Asn Tyr Lys Val
50 55 60

Gln Val Lys Ile Gln Asn Pro Thr Ala Cys Thr Ala Asn Gly Ser Cys
65 70 75 80

Asp Pro Ser Val Thr Arg Gln Lys Tyr Ala Asp Val Thr Phe Ser Phe
85 90 95

Thr Gln Tyr Ser Thr Asp Glu Glu Arg Ala Phe Val Arg Thr Glu Leu
100 105 110

Ala Ala Leu Leu Ala Ser Pro Leu Leu Ile Asp Ala Ile Asp Gln Leu
115 120 125

Asn Pro Ala Tyr
130

<210> 10
<211> 132
<212> PRT
<213> Bacteriophage Qbeta

<400> 10

Ala Lys Leu Glu Thr Val Thr Leu Gly Asn Ile Gly Lys Asp Gly Arg
1 5 10 15

Gln Thr Leu Val Leu Asn Pro Arg Gly Val Asn Pro Thr Asn Gly Val
20 25 30

Ala Ser Leu Ser Gln Ala Gly Ala Val Pro Ala Leu Glu Lys Arg Val
35 40 45

Thr Val Ser Val Ser Gln Pro Ser Arg Asn Arg Lys Asn Tyr Lys Val
50 55 60

Gln Val Lys Ile Gln Asn Pro Thr Ala Cys Thr Ala Asn Gly Ser Cys
65 70 75 80

Asp Pro Ser Val Thr Arg Gln Lys Tyr Ala Asp Val Thr Phe Ser Phe

85 90 95

Thr Gln Tyr Ser Thr Asp Glu Glu Arg Ala Phe Val Arg Thr Glu Leu
100 105 110

Ala Ala Leu Leu Ala Ser Pro Leu Leu Ile Asp Ala Ile Asp Gln Leu
115 120 125

Asn Pro Ala Tyr
130

<210> 11
<211> 132
<212> PRT
<213> Bacteriophage Qbeta

<400> 11

Ala Arg Leu Glu Thr Val Thr Leu Gly Asn Ile Gly Lys Asp Gly Arg
1 5 10 15

Gln Thr Leu Val Leu Asn Pro Arg Gly Val Asn Pro Thr Asn Gly Val
20 25 30

Ala Ser Leu Ser Gln Ala Gly Ala Val Pro Ala Leu Glu Lys Arg Val
35 40 45

Thr Val Ser Val Ser Gln Pro Ser Arg Asn Arg Lys Asn Tyr Lys Val
50 55 60

Gln Val Lys Ile Gln Asn Pro Thr Ala Cys Thr Ala Asn Gly Ser Cys
65 70 75 80

Asp Pro Ser Val Thr Arg Gln Lys Tyr Ala Asp Val Thr Phe Ser Phe
85 90 95

Thr Gln Tyr Ser Thr Asp Glu Glu Arg Ala Phe Val Arg Thr Glu Leu
100 105 110

Ala Ala Leu Leu Ala Ser Pro Leu Leu Ile Asp Ala Ile Asp Gln Leu
115 120 125

Asn Pro Ala Tyr
130

<210> 12
<211> 131
<212> PRT
<213> Bacteriophage AP205

<400> 12

Met Ala Asn Lys Pro Met Gln Pro Ile Thr Ser Thr Ala Asn Lys Ile
1 5 10 15

Val Trp Ser Asp Pro Thr Arg Leu Ser Thr Thr Phe Ser Ala Ser Leu
20 25 30

Leu Arg Gln Arg Val Lys Val Gly Ile Ala Glu Leu Asn Asn Val Ser
35 40 45

Gly Gln Tyr Val Ser Val Tyr Lys Arg Pro Ala Pro Lys Pro Glu Gly
50 55 60

Cys Ala Asp Ala Cys Val Ile Met Pro Asn Glu Asn Gln Ser Ile Arg
65 70 75 80

Thr Val Ile Ser Gly Ser Ala Glu Asn Leu Ala Thr Leu Lys Ala Glu
85 90 95

Trp Glu Thr His Lys Arg Asn Val Asp Thr Leu Phe Ala Ser Gly Asn
100 105 110

Ala Gly Leu Gly Phe Leu Asp Pro Thr Ala Ala Ile Val Ser Ser Asp
115 120 125

Thr Thr Ala
130

<210> 13
<211> 131
<212> PRT
<213> Bacteriophage AP205

<400> 13

Met Ala Asn Lys Thr Met Gln Pro Ile Thr Ser Thr Ala Asn Lys Ile
1 5 10 15

Val Trp Ser Asp Pro Thr Arg Leu Ser Thr Thr Phe Ser Ala Ser Leu
20 25 30

Leu Arg Gln Arg Val Lys Val Gly Ile Ala Glu Leu Asn Asn Val Ser
35 40 45

Gly Gln Tyr Val Ser Val Tyr Lys Arg Pro Ala Pro Lys Pro Glu Gly
50 55 60

```
Cys Ala Asp Ala Cys Val Ile Met Pro Asn Glu Asn Gln Ser Ile Arg
65                  70                  75                  80

Thr Val Ile Ser Gly Ser Ala Glu Asn Leu Ala Thr Leu Lys Ala Glu
                85                  90                  95

Trp Glu Thr His Lys Arg Asn Val Asp Thr Leu Phe Ala Ser Gly Asn
            100                 105                 110

Ala Gly Leu Gly Phe Leu Asp Pro Thr Ala Ala Ile Val Ser Ser Asp
        115                 120                 125

Thr Thr Ala
    130


<210>  14
<211>  131
<212>  PRT
<213>  Bacteriophage AP205

<400>  14

Met Ala Asn Lys Pro Met Gln Pro Ile Thr Ser Thr Ala Asp Lys Ile
1               5                   10                  15

Val Trp Ser Asp Pro Thr Arg Leu Ser Thr Thr Phe Ser Ala Ser Leu
            20                  25                  30

Leu Arg Gln Arg Val Lys Val Gly Ile Ala Glu Leu Asn Asn Val Ser
        35                  40                  45

Gly Gln Tyr Val Ser Val Tyr Lys Arg Pro Ala Pro Lys Pro Glu Gly
    50                  55                  60

Cys Ala Asp Ala Cys Val Ile Met Pro Asn Glu Asn Gln Ser Ile Arg
65                  70                  75                  80

Thr Val Ile Ser Gly Ser Ala Glu Asn Leu Ala Thr Leu Lys Ala Glu
                85                  90                  95

Trp Glu Thr His Lys Arg Asn Val Asp Thr Leu Phe Ala Ser Gly Asn
            100                 105                 110

Ala Gly Leu Gly Phe Leu Asp Pro Thr Ala Ala Ile Val Ser Ser Asp
        115                 120                 125

Thr Thr Ala
    130
```

<210> 15
<211> 329
<212> PRT
<213> Bacteriophage Qbeta

<400> 15

```
Met Ala Lys Leu Glu Thr Val Thr Leu Gly Asn Ile Gly Lys Asp Gly
1               5                   10                  15

Lys Gln Thr Leu Val Leu Asn Pro Arg Gly Val Asn Pro Thr Asn Gly
            20                  25                  30

Val Ala Ser Leu Ser Gln Ala Gly Ala Val Pro Ala Leu Glu Lys Arg
        35                  40                  45

Val Thr Val Ser Val Ser Gln Pro Ser Arg Asn Arg Lys Asn Tyr Lys
    50                  55                  60

Val Gln Val Lys Ile Gln Asn Pro Thr Ala Cys Thr Ala Asn Gly Ser
65                  70                  75                  80

Cys Asp Pro Ser Val Thr Arg Gln Ala Tyr Ala Asp Val Thr Phe Ser
                85                  90                  95

Phe Thr Gln Tyr Ser Thr Asp Glu Glu Arg Ala Phe Val Arg Thr Glu
            100                 105                 110

Leu Ala Ala Leu Leu Ala Ser Pro Leu Leu Ile Asp Ala Ile Asp Gln
        115                 120                 125

Leu Asn Pro Ala Tyr Trp Thr Leu Leu Ile Ala Gly Gly Gly Ser Gly
    130                 135                 140

Ser Lys Pro Asp Pro Val Ile Pro Asp Pro Pro Ile Asp Pro Pro Pro
145                 150                 155                 160

Gly Thr Gly Lys Tyr Thr Cys Pro Phe Ala Ile Trp Ser Leu Glu Glu
                165                 170                 175

Val Tyr Glu Pro Pro Thr Lys Asn Arg Pro Trp Pro Ile Tyr Asn Ala
            180                 185                 190

Val Glu Leu Gln Pro Arg Glu Phe Asp Val Ala Leu Lys Asp Leu Leu
        195                 200                 205

Gly Asn Thr Lys Trp Arg Asp Trp Asp Ser Arg Leu Ser Tyr Thr Thr
    210                 215                 220
```

Phe Arg Gly Cys Arg Gly Asn Gly Tyr Ile Asp Leu Asp Ala Thr Tyr
225 230 235 240

Leu Ala Thr Asp Gln Ala Met Arg Asp Gln Lys Tyr Asp Ile Arg Glu
245 250 255

Gly Lys Lys Pro Gly Ala Phe Gly Asn Ile Glu Arg Phe Ile Tyr Leu
260 265 270

Lys Ser Ile Asn Ala Tyr Cys Ser Leu Ser Asp Ile Ala Ala Tyr His
275 280 285

Ala Asp Gly Val Ile Val Gly Phe Trp Arg Asp Pro Ser Ser Gly Gly
290 295 300

Ala Ile Pro Phe Asp Phe Thr Lys Phe Asp Lys Thr Lys Cys Pro Ile
305 310 315 320

Gln Ala Val Ile Val Val Pro Arg Ala
325

<210> 16
<211> 129
<212> PRT
<213> Bacteriophage R17

<400> 16

Ala Ser Asn Phe Thr Gln Phe Val Leu Val Asn Asp Gly Gly Thr Gly
1 5 10 15

Asn Val Thr Val Ala Pro Ser Asn Phe Ala Asn Gly Val Ala Glu Trp
20 25 30

Ile Ser Ser Asn Ser Arg Ser Gln Ala Tyr Lys Val Thr Cys Ser Val
35 40 45

Arg Gln Ser Ser Ala Gln Asn Arg Lys Tyr Thr Ile Lys Val Glu Val
50 55 60

Pro Lys Val Ala Thr Gln Thr Val Gly Gly Val Glu Leu Pro Val Ala
65 70 75 80

Ala Trp Arg Ser Tyr Leu Asn Met Glu Leu Thr Ile Pro Ile Phe Ala
85 90 95

Thr Asn Ser Asp Cys Glu Leu Ile Val Lys Ala Met Gln Gly Leu Leu

100 105 110

Lys Asp Gly Asn Pro Ile Pro Ser Ala Ile Ala Ala Asn Ser Gly Ile
115 120 125

Tyr

<210> 17
<211> 130
<212> PRT
<213> Bacteriophage fr

<400> 17

Met Ala Ser Asn Phe Glu Glu Phe Val Leu Val Asp Asn Gly Gly Thr
1 5 10 15

Gly Asp Val Lys Val Ala Pro Ser Asn Phe Ala Asn Gly Val Ala Glu
20 25 30

Trp Ile Ser Ser Asn Ser Arg Ser Gln Ala Tyr Lys Val Thr Cys Ser
35 40 45

Val Arg Gln Ser Ser Ala Asn Asn Arg Lys Tyr Thr Val Lys Val Glu
50 55 60

Val Pro Lys Val Ala Thr Gln Val Gln Gly Gly Val Glu Leu Pro Val
65 70 75 80

Ala Ala Trp Arg Ser Tyr Met Asn Met Glu Leu Thr Ile Pro Val Phe
85 90 95

Ala Thr Asn Asp Asp Cys Ala Leu Ile Val Lys Ala Leu Gln Gly Thr
100 105 110

Phe Lys Thr Gly Asn Pro Ile Ala Thr Ala Ile Ala Ala Asn Ser Gly
115 120 125

Ile Tyr
130

<210> 18
<211> 130
<212> PRT
<213> Bacteriophage GA

<400> 18

Met Ala Thr Leu Arg Ser Phe Val Leu Val Asp Asn Gly Gly Thr Gly

1 5 10 15

Asn Val Thr Val Val Pro Val Ser Asn Ala Asn Gly Val Ala Glu Trp
20 25 30

Leu Ser Asn Asn Ser Arg Ser Gln Ala Tyr Arg Val Thr Ala Ser Tyr
35 40 45

Arg Ala Ser Gly Ala Asp Lys Arg Lys Tyr Ala Ile Lys Leu Glu Val
50 55 60

Pro Lys Ile Val Thr Gln Val Val Asn Gly Val Glu Leu Pro Gly Ser
65 70 75 80

Ala Trp Lys Ala Tyr Ala Ser Ile Asp Leu Thr Ile Pro Ile Phe Ala
85 90 95

Ala Thr Asp Asp Val Thr Val Ile Ser Lys Ser Leu Ala Gly Leu Phe
100 105 110

Lys Val Gly Asn Pro Ile Ala Glu Ala Ile Ser Ser Gln Ser Gly Phe
115 120 125

Tyr Ala
130

<210> 19
<211> 132
<212> PRT
<213> Bacteriophage SP

<400> 19

Met Ala Lys Leu Asn Gln Val Thr Leu Ser Lys Ile Gly Lys Asn Gly
1 5 10 15

Asp Gln Thr Leu Thr Leu Thr Pro Arg Gly Val Asn Pro Thr Asn Gly
20 25 30

Val Ala Ser Leu Ser Glu Ala Gly Ala Val Pro Ala Leu Glu Lys Arg
35 40 45

Val Thr Val Ser Val Ala Gln Pro Ser Arg Asn Arg Lys Asn Phe Lys
50 55 60

Val Gln Ile Lys Leu Gln Asn Pro Thr Ala Cys Thr Arg Asp Ala Cys
65 70 75 80

Asp Pro Ser Val Thr Arg Ser Ala Phe Ala Asp Val Thr Leu Ser Phe
85 90 95

Thr Ser Tyr Ser Thr Asp Glu Glu Arg Ala Leu Ile Arg Thr Glu Leu
100 105 110

Ala Ala Leu Leu Ala Asp Pro Leu Ile Val Asp Ala Ile Asp Asn Leu
115 120 125

Asn Pro Ala Tyr
130

<210> 20
<211> 329
<212> PRT
<213> Bacteriophage

<400> 20

Ala Lys Leu Asn Gln Val Thr Leu Ser Lys Ile Gly Lys Asn Gly Asp
1 5 10 15

Gln Thr Leu Thr Leu Thr Pro Arg Gly Val Asn Pro Thr Asn Gly Val
20 25 30

Ala Ser Leu Ser Glu Ala Gly Ala Val Pro Ala Leu Glu Lys Arg Val
35 40 45

Thr Val Ser Val Ala Gln Pro Ser Arg Asn Arg Lys Asn Phe Lys Val
50 55 60

Gln Ile Lys Leu Gln Asn Pro Thr Ala Cys Thr Arg Asp Ala Cys Asp
65 70 75 80

Pro Ser Val Thr Arg Ser Ala Phe Ala Asp Val Thr Leu Ser Phe Thr
85 90 95

Ser Tyr Ser Thr Asp Glu Glu Arg Ala Leu Ile Arg Thr Glu Leu Ala
100 105 110

Ala Leu Leu Ala Asp Pro Leu Ile Val Asp Ala Ile Asp Asn Leu Asn
115 120 125

Pro Ala Tyr Trp Ala Ala Leu Leu Val Ala Ser Ser Gly Gly Gly Asp
130 135 140

Asn Pro Ser Asp Pro Asp Val Pro Val Val Pro Asp Val Lys Pro Pro
145 150 155 160

```
Asp Gly Thr Gly Arg Tyr Lys Cys Pro Phe Ala Cys Tyr Arg Leu Gly
                165                 170                 175

Ser Ile Tyr Glu Val Gly Lys Glu Gly Ser Pro Asp Ile Tyr Glu Arg
            180                 185                 190

Gly Asp Glu Val Ser Val Thr Phe Asp Tyr Ala Leu Glu Asp Phe Leu
        195                 200                 205

Gly Asn Thr Asn Trp Arg Asn Trp Asp Gln Arg Leu Ser Asp Tyr Asp
    210                 215                 220

Ile Ala Asn Arg Arg Arg Cys Arg Gly Asn Gly Tyr Ile Asp Leu Asp
225                 230                 235                 240

Ala Thr Ala Met Gln Ser Asp Asp Phe Val Leu Ser Gly Arg Tyr Gly
                245                 250                 255

Val Arg Lys Val Lys Phe Pro Gly Ala Phe Gly Ser Ile Lys Tyr Leu
            260                 265                 270

Leu Asn Ile Gln Gly Asp Ala Trp Leu Asp Leu Ser Glu Val Thr Ala
        275                 280                 285

Tyr Arg Ser Tyr Gly Met Val Ile Gly Phe Trp Thr Asp Ser Lys Ser
    290                 295                 300

Pro Gln Leu Pro Thr Asp Phe Thr Gln Phe Asn Ser Ala Asn Cys Pro
305                 310                 315                 320

Val Gln Thr Val Ile Ile Ile Pro Ser
                325
```

<210>  21
<211>  130
<212>  PRT
<213>  Bacteriophage MS2

<400>  21

```
Met Ala Ser Asn Phe Thr Gln Phe Val Leu Val Asp Asn Gly Gly Thr
1               5                   10                  15

Gly Asp Val Thr Val Ala Pro Ser Asn Phe Ala Asn Gly Val Ala Glu
            20                  25                  30

Trp Ile Ser Ser Asn Ser Arg Ser Gln Ala Tyr Lys Val Thr Cys Ser
        35                  40                  45
```

Val Arg Gln Ser Ser Ala Gln Asn Arg Lys Tyr Thr Ile Lys Val Glu
50 55 60

Val Pro Lys Val Ala Thr Gln Thr Val Gly Gly Val Glu Leu Pro Val
65 70 75 80

Ala Ala Trp Arg Ser Tyr Leu Asn Met Glu Leu Thr Ile Pro Ile Phe
85 90 95

Ala Thr Asn Ser Asp Cys Glu Leu Ile Val Lys Ala Met Gln Gly Leu
100 105 110

Leu Lys Asp Gly Asn Pro Ile Pro Ser Ala Ile Ala Ala Asn Ser Gly
115 120 125

Ile Tyr
130

<210> 22
<211> 133
<212> PRT
<213> Bacteriophage M11

<400> 22

Met Ala Lys Leu Gln Ala Ile Thr Leu Ser Gly Ile Gly Lys Lys Gly
1 5 10 15

Asp Val Thr Leu Asp Leu Asn Pro Arg Gly Val Asn Pro Thr Asn Gly
20 25 30

Val Ala Ala Leu Ser Glu Ala Gly Ala Val Pro Ala Leu Glu Lys Arg
35 40 45

Val Thr Ile Ser Val Ser Gln Pro Ser Arg Asn Arg Lys Asn Tyr Lys
50 55 60

Val Gln Val Lys Ile Gln Asn Pro Thr Ser Cys Thr Ala Ser Gly Thr
65 70 75 80

Cys Asp Pro Ser Val Thr Arg Ser Ala Tyr Ser Asp Val Thr Phe Ser
85 90 95

Phe Thr Gln Tyr Ser Thr Val Glu Glu Arg Ala Leu Val Arg Thr Glu
100 105 110

Leu Gln Ala Leu Leu Ala Asp Pro Met Leu Val Asn Ala Ile Asp Asn

115 120 125

Leu Asn Pro Ala Tyr
130

<210> 23
<211> 133
<212> PRT
<213> Bacteriophage MX1

<400> 23

Met Ala Lys Leu Gln Ala Ile Thr Leu Ser Gly Ile Gly Lys Asn Gly
1 5 10 15

Asp Val Thr Leu Asn Leu Asn Pro Arg Gly Val Asn Pro Thr Asn Gly
20 25 30

Val Ala Ala Leu Ser Glu Ala Gly Ala Val Pro Ala Leu Glu Lys Arg
35 40 45

Val Thr Ile Ser Val Ser Gln Pro Ser Arg Asn Arg Lys Asn Tyr Lys
50 55 60

Val Gln Val Lys Ile Gln Asn Pro Thr Ser Cys Thr Ala Ser Gly Thr
65 70 75 80

Cys Asp Pro Ser Val Thr Arg Ser Ala Tyr Ala Asp Val Thr Phe Ser
85 90 95

Phe Thr Gln Tyr Ser Thr Asp Glu Glu Arg Ala Leu Val Arg Thr Glu
100 105 110

Leu Lys Ala Leu Leu Ala Asp Pro Met Leu Ile Asp Ala Ile Asp Asn
115 120 125

Leu Asn Pro Ala Tyr
130

<210> 24
<211> 330
<212> PRT
<213> Bacteriophage NL95

<400> 24

Met Ala Lys Leu Asn Lys Val Thr Leu Thr Gly Ile Gly Lys Ala Gly
1 5 10 15

Asn Gln Thr Leu Thr Leu Thr Pro Arg Gly Val Asn Pro Thr Asn Gly

```
            20                  25                  30

Val Ala Ser Leu Ser Glu Ala Gly Ala Val Pro Ala Leu Glu Lys Arg
        35                  40                  45

Val Thr Val Ser Val Ala Gln Pro Ser Arg Asn Arg Lys Asn Tyr Lys
    50                  55                  60

Val Gln Ile Lys Leu Gln Asn Pro Thr Ala Cys Thr Lys Asp Ala Cys
65                  70                  75                  80

Asp Pro Ser Val Thr Arg Ser Gly Ser Arg Asp Val Thr Leu Ser Phe
                85                  90                  95

Thr Ser Tyr Ser Thr Glu Arg Glu Arg Ala Leu Ile Arg Thr Glu Leu
            100                 105                 110

Ala Ala Leu Leu Lys Asp Asp Leu Ile Val Asp Ala Ile Asp Asn Leu
        115                 120                 125

Asn Pro Ala Tyr Trp Ala Ala Leu Leu Ala Ala Ser Pro Gly Gly Gly
    130                 135                 140

Asn Asn Pro Tyr Pro Gly Val Pro Asp Ser Pro Asn Val Lys Pro Pro
145                 150                 155                 160

Gly Gly Thr Gly Thr Tyr Arg Cys Pro Phe Ala Cys Tyr Arg Arg Gly
                165                 170                 175

Glu Leu Ile Thr Glu Ala Lys Asp Gly Ala Cys Ala Leu Tyr Ala Cys
            180                 185                 190

Gly Ser Glu Ala Leu Val Glu Phe Glu Tyr Ala Leu Glu Asp Phe Leu
        195                 200                 205

Gly Asn Glu Phe Trp Arg Asn Trp Asp Gly Arg Leu Ser Lys Tyr Asp
    210                 215                 220

Ile Glu Thr His Arg Arg Cys Arg Gly Asn Gly Tyr Val Asp Leu Asp
225                 230                 235                 240

Ala Ser Val Met Gln Ser Asp Glu Tyr Val Leu Ser Gly Ala Tyr Asp
                245                 250                 255

Val Val Lys Met Gln Pro Pro Gly Thr Phe Asp Ser Pro Arg Tyr Tyr
            260                 265                 270
```

Leu His Leu Met Asp Gly Ile Tyr Val Asp Leu Ala Glu Val Thr Ala
        275                 280                 285

Tyr Arg Ser Tyr Gly Met Val Ile Gly Phe Trp Thr Asp Ser Lys Ser
    290                 295                 300

Pro Gln Leu Pro Thr Asp Phe Thr Arg Phe Asn Arg His Asn Cys Pro
305                 310                 315                 320

Val Gln Thr Val Ile Val Ile Pro Ser Leu
                325                 330

<210> 25
<211> 129
<212> PRT
<213> Bacteriophage f2

<400> 25

Ala Ser Asn Phe Thr Gln Phe Val Leu Val Asn Asp Gly Gly Thr Gly
1               5                   10                  15

Asn Val Thr Val Ala Pro Ser Asn Phe Ala Asn Gly Val Ala Glu Trp
            20                  25                  30

Ile Ser Ser Asn Ser Arg Ser Gln Ala Tyr Lys Val Thr Cys Ser Val
        35                  40                  45

Arg Gln Ser Ser Ala Gln Asn Arg Lys Tyr Thr Ile Lys Val Glu Val
    50                  55                  60

Pro Lys Val Ala Thr Gln Thr Val Gly Gly Val Glu Leu Pro Val Ala
65                  70                  75                  80

Ala Trp Arg Ser Tyr Leu Asn Leu Glu Leu Thr Ile Pro Ile Phe Ala
                85                  90                  95

Thr Asn Ser Asp Cys Glu Leu Ile Val Lys Ala Met Gln Gly Leu Leu
            100                 105                 110

Lys Asp Gly Asn Pro Ile Pro Ser Ala Ile Ala Ala Asn Ser Gly Ile
        115                 120                 125

Tyr

<210> 26
<211> 128

<212> PRT
<213> Bacteriophage PP7

<400> 26

Met Ser Lys Thr Ile Val Leu Ser Val Gly Glu Ala Thr Arg Thr Leu
1 5 10 15

Thr Glu Ile Gln Ser Thr Ala Asp Arg Gln Ile Phe Glu Glu Lys Val
20 25 30

Gly Pro Leu Val Gly Arg Leu Arg Leu Thr Ala Ser Leu Arg Gln Asn
35 40 45

Gly Ala Lys Thr Ala Tyr Arg Val Asn Leu Lys Leu Asp Gln Ala Asp
50 55 60

Val Val Asp Cys Ser Thr Ser Val Cys Gly Glu Leu Pro Lys Val Arg
65 70 75 80

Tyr Thr Gln Val Trp Ser His Asp Val Thr Ile Val Ala Asn Ser Thr
85 90 95

Glu Ala Ser Arg Lys Ser Leu Tyr Asp Leu Thr Lys Ser Leu Val Ala
100 105 110

Thr Ser Gln Val Glu Asp Leu Val Val Asn Leu Val Pro Leu Gly Arg
115 120 125

<210> 27
<211> 4586
<212> DNA
<213> artificial sequence

<220>
<223> Cloning Vector

<400> 27
ttctgtttcc tgtgtgaaat tgttatccgc tcacaattcc acacattata cgagccgatg 60

attaattgtc aacagctcat ttcagaatat ttgccagaac cgttatgatg tcggcgcaaa 120

aaacattatc cagaacggga gtgcgccttg agcgacacga attatgcagt gatttacgac 180

ctgcacagcc ataccacagc ttccgatggc tgcctgacgc cagaagcatt ggtgcaccgt 240

gcagtcgata agctccggat cctctacgcc ggacgcatcg tggccggcat caccggcgcc 300

acaggtgcgg ttgctggcgc ctatatcgcc gacatcaccg atggggaaga tcgggctcgc 360

cacttcgggc tcatgagcgc ttgtttcggc gtgggtatgg tggcaggccc cgtggccggg 420

ggactgttgg gcgccatctc cttgcatgca ccattccttg cggcggcggt gctcaacggc 480

```
ctcaacctac tactgggctg cttcctaatg caggagtcgc ataagggaga gcgtcgaccg     540
atgcccttga gagccttcaa cccagtcagc tccttccggt gggcgcgggg catgactatc     600
gtcgccgcac ttatgactgt cttctttatc atgcaactcg taggacaggt gccggcagcg     660
ctctgggtca ttttcggcga ggaccgcttt cgctggagcg cgacgatgat cggcctgtcg     720
cttgcggtat tcggaatctt gcacgccctc gctcaagcct tcgtcactgg tcccgccacc     780
aaacgtttcg gcgagaagca ggccattatc gccggcatgg cggccgacgc gctgggctac     840
gtcttgctgg cgttcgcgac gcgaggctgg atggccttcc ccattatgat tcttctcgct     900
tccggcggca tcgggatgcc cgcgttgcag gccatgctgt ccaggcaggt agatgacgac     960
catcagggac agcttcaagg atcgctcgcg gctcttacca gcctaacttc gatcactgga    1020
ccgctgatcg tcacggcgat ttatgccgcc tcggcgagca catggaacgg gttggcatgg    1080
attgtaggcg ccgccctata ccttgtctgc ctccccgcgt tgcgtcgcgg tgcatggagc    1140
cgggccacct cgacctgaat ggaagccggc ggcacctcgc taacggattc accactccaa    1200
gaattggagc caatcaattc ttgcggagaa ctgtgaatgc gcaaaccaac ccttggcaga    1260
acatatccat cgcgtccgcc atctccagca gccgcacgcg gcgcatctcg ggcagcgttg    1320
ggtcctggcc acgggtgcgc atgatcgtgc tcctgtcgtt gaggacccgg ctaggctggc    1380
ggggttgcct tactggttag cagaatgaat caccgatacg cgagcgaacg tgaagcgact    1440
gctgctgcaa aacgtctgcg acctgagcaa caacatgaat ggtcttcggt ttccgtgttt    1500
cgtaaagtct ggaaacgcgg aagtcagcgc cctgcaccat tatgttccgg atctgcatcg    1560
caggatgctg ctggctaccc tgtggaacac ctacatctgt attaacgaag cgctggcatt    1620
gaccctgagt gatttttctc tggtcccgcc gcatccatac cgccagttgt ttaccctcac    1680
aacgttccag taaccgggca tgttcatcat cagtaacccg tatcgtgagc atcctctctc    1740
gtttcatcgg tatcattacc cccatgaaca gaaattcccc cttacacgga ggcatcaagt    1800
gaccaaacag gaaaaaaccg cccttaacat ggcccgcttt atcagaagcc agacattaac    1860
gcttctggag aaactcaacg agctggacgc ggatgaacag gcagacatct gtgaatcgct    1920
tcacgaccac gctgatgagc tttaccgcag ctgcctcgcg cgtttcggtg atgacggtga    1980
aaacctctga cacatgcagc tcccggagac ggtcacagct tgtctgtaag cggatgccgg    2040
gagcagacaa gcccgtcagg gcgcgtcagc gggtgttggc gggtgtcggg gcgcagccat    2100
gacccagtca cgtagcgata gcggagtgta tactggctta actatgcggc atcagagcag    2160
attgtactga gagtgcacca tatgcggtgt gaaataccgc acagatgcgt aaggagaaaa    2220
taccgcatca ggcgctcttc cgcttcctcg ctcactgact cgctgcgctc ggtcgttcgg    2280
ctgcggcgag cggtatcagc tcactcaaag gcggtaatac ggttatccac agaatcaggg    2340
```

gataacgcag gaaagaacat gtgagcaaaa ggccagcaaa aggccaggaa ccgtaaaaag 2400

gccgcgttgc tggcgttttt ccataggctc cgcccccctg acgagcatca caaaaatcga 2460

cgctcaagtc agaggtggcg aaacccgaca ggactataaa gataccaggc gtttccccct 2520

ggaagctccc tcgtgcgctc tcctgttccg accctgccgc ttaccggata cctgtccgcc 2580

tttctccctt cgggaagcgt ggcgctttct catagctcac gctgtaggta tctcagttcg 2640

gtgtaggtcg ttcgctccaa gctgggctgt gtgcacgaac cccccgttca gcccgaccgc 2700

tgcgccttat ccggtaacta tcgtcttgag tccaacccgg taagacacga cttatcgcca 2760

ctggcagcag ccactggtaa caggattagc agagcgaggt atgtaggcgg tgctacagag 2820

ttcttgaagt ggtggcctaa ctacggctac actagaagga cagtatttgg tatctgcgct 2880

ctgctgaagc cagttacctt cggaaaaaga gttggtagct cttgatccgg caaacaaacc 2940

accgctggta gcggtggttt ttttgtttgc aagcagcaga ttacgcgcag aaaaaaagga 3000

tctcaagaag atcctttgat cttttctacg gggtctgacg ctcagtggaa cgaaaactca 3060

cgttaaggga ttttggtcat gagattatca aaaaggatct tcacctagat ccttttaaat 3120

taaaaatgaa gttttaaatc aatctaaagt atatatgagt aaacttggtc tgacagttac 3180

caatgcttaa tcagtgaggc acctatctca gcgatctgtc tatttcgttc atccatagtt 3240

gcctgactcc ccgtcgtgta gataactacg atacgggagg gcttaccatc tggccccagt 3300

gctgcaatga taccgcgaga cccacgctca ccggctccag atttatcagc aataaaccag 3360

ccagccggaa gggccgagcg cagaagtggt cctgcaactt tatccgcctc catccagtct 3420

attaattgtt gccgggaagc tagagtaagt agttcgccag ttaatagttt gcgcaacgtt 3480

gttgccattg ctacaggcat cgtggtgtca cgctcgtcgt ttggtatggc ttcattcagc 3540

tccggttccc aacgatcaag gcgagttaca tgatccccca tgttgtgcaa aaaagcggtt 3600

agctccttcg gtcctccgat cgttgtcaga agtaagttgg ccgcagtgtt atcactcatg 3660

gttatggcag cactgcataa ttctcttact gtcatgccat ccgtaagatg cttttctgtg 3720

actggtgagt actcaaccaa gtcattctga gaatagtgta tgcggcgacc gagttgctct 3780

tgcccggcgt caacacggga taataccgcg ccacatagca gaactttaaa agtgctcatc 3840

attggaaaac gttcttcggg gcgaaaactc tcaaggatct taccgctgtt gagatccagt 3900

tcgatgtaac ccactcgtgc acccaactga tcttcagcat cttttacttt caccagcgtt 3960

tctgggtgag caaaaacagg aaggcaaaat gccgcaaaaa agggaataag ggcgacacgg 4020

aaatgttgaa tactcatact cttccttttt caatattatt gaagcattta tcagggttat 4080

tgtctcatga gcggatacat atttgaatgt atttagaaaa ataaacaaaa gagtttgtag 4140

aaacgcaaaa aggccatccg tcaggatggc cttctgctta atttgatgcc tggcagttta 4200

tggcgggcgt cctgcccgcc accctccggg ccgttgcttc gcaacgttca aatccgctcc 4260

cggcggattt gtcctactca ggagagcgtt caccgacaaa caacagataa aacgaaaggc 4320

ccagtctttc gactgagcct ttcgttttat ttgatgcctg gcagttccct actctcgcat 4380

ggggagaccc cacactacca tcggcgctac ggcgtttcac ttctgagttc ggcatggggt 4440

caggtgggac caccgcgcta ctgccgccag gcaaattctg ttttatcaga ccgcttctgc 4500

gttctgattt aatctgtatc aggctgaaaa tcttctctca tccgccaaaa cagaagcttg 4560

gctgcaggtc gacggatccc cgggaa 4586

<210> 28
<211> 425
<212> DNA
<213> artificial sequence

<220>
<223> Terminator Sequence

<400> 28
ctgttttggc ggatgagaga agattttcag cctgatacag attaaatcag aacgcagaag 60

cggtctgata aaacagaatt tgcctggcgg cagtagcgcg gtggtcccac ctgaccccat 120

gccgaactca gaagtgaaac gccgtagcgc cgatggtagt gtggggtctc cccatgcgag 180

agtagggaac tgccaggcat caaataaaac gaaaggctca gtcgaaagac tgggcctttc 240

gttttatctg ttgtttgtcg gtgaacgctc tcctgagtag gacaaatccg ccgggagcgg 300

atttgaacgt tgcgaagcaa cggcccggag ggtggcgggc aggacgcccg ccataaactg 360

ccaggcatca aattaagcag aaggccatcc tgacggatgg cctttttgcg tttctacaaa 420

ctctt 425

<210> 29
<211> 816
<212> DNA
<213> artificial sequence

<220>
<223> Resistance gene

<400> 29
ttagaaaaac tcatcgagca tcaaatgaaa ctgcaattta ttcatatcag gattatcaat 60

accatatttt tgaaaaagcc gtttctgtaa tgaaggagaa aactcaccga ggcagttcca 120

taggatggca agatcctggt atcggtctgc gattccgact cgtccaacat caatacaacc 180

tattaatttc ccctcgtcaa aaataaggtt atcaagtgag aaatcaccat gagtgacgac 240

tgaatccggt gagaatggca aaagcttatg catttctttc cagacttgtt caacaggcca 300

gccattacgc tcgtcatcaa aatcactcgc atcaaccaaa ccgttattca ttcgtgattg 360

cgcctgagcg agacgaaata cgcgatcgct gttaaaagga caattacaaa caggaatcga 420

atgcaaccgg cgcaggaaca ctgccagcgc atcaacaata ttttcacctg aatcaggata 480

ttcttctaat acctggaatg ctgttttccc ggggatcgca gtggtgagta accatgcatc 540

atcaggagta cggataaaat gcttgatggt cggaagaggc ataaattccg tcagccagtt 600

tagtctgacc atctcatctg taacatcatt ggcaacgcta cctttgccat gtttcagaaa 660

caactctggc gcatcgggct tcccatacaa tcgatagatt gtcgcacctg attgcccgac 720

attatcgcga gcccatttat acccatataa atcagcatcc atgttggaat ttaatcgcgg 780

cctcgagcaa gacgtttccc gttgaatatg gctcat 816

<210> 30
<211> 4963
<212> DNA
<213> artificial sequence

<220>
<223> Plasmid

<400> 30
ggctgtgcag gtcgtaaatc actgcataat tcgtgtcgct caaggcgcac tcccgttctg 60

gataatgttt tttgcgccga catcataacg gttctggcaa atattctgaa atgagctgtt 120

gacaattaat catcggctcg tataatgtgt ggaattgtga gcggataaca atttcacaca 180

ggaaacagaa ttctaaggag gaaaaaaaaa tggcaaataa gccaatgcaa ccgatcacat 240

ctacagcaaa taaaattgtg tggtcggatc caactcgttt atcaactaca ttttcagcaa 300

gtctgttacg ccaacgtgtt aaagttggta tagccgaact gaataatgtt tcaggtcaat 360

atgtatctgt ttataagcgt cctgcaccta aaccggaagg ttgtgcagat gcctgtgtca 420

ttatgccgaa tgaaaaccaa tccattcgca cagtgatttc agggtcagcc gaaaacttgg 480

ctaccttaaa agcagaatgg gaaactcaca aacgtaacgt tgacacactc ttcgcgagcg 540

gcaacgccgg tttgggtttc cttgacccta ctgcggctat cgtatcgtct gatactactg 600

cttaatgaag cttggctgtt ttggcggatg agagaagatt ttcagcctga tacagattaa 660

atcagaacgc agaagcggtc tgataaaaca gaatttgcct ggcggcagta gcgcggtggt 720

cccacctgac cccatgccga actcagaagt gaaacgccgt agcgccgatg gtagtgtggg 780

gtctccccat gcgagagtag ggaactgcca ggcatcaaat aaaacgaaag gctcagtcga 840

aagactgggc ctttcgtttt atctgttgtt tgtcggtgaa cgctctcctg agtaggacaa 900

atccgccggg agcggatttg aacgttgcga agcaacggcc cggagggtgg cgggcaggac 960

gcccgccata aactgccagg catcaaatta agcagaaggc catcctgacg gatggccttt 1020

ttgcgtttct acaaactctt ttgtttattt ttctagagcc acgttgtgtc tcaaaatctc 1080

tgatgttaca ttgcacaaga taaaaatata tcatcatgaa caataaaact gtctgcttac 1140
ataaacagta atacaaggag tgttatgagc catattcaac gggaaacgtc ttgctcgagg 1200
ccgcgattaa attccaacat ggatgctgat ttatatgggt ataaatgggc tcgcgataat 1260
gtcgggcaat caggtgcgac aatctatcga ttgtatggga agcccgatgc gccagagttg 1320
tttctgaaac atggcaaagg tagcgttgcc aatgatgtta cagatgagat ggtcagacta 1380
aactggctga cggaatttat gcctcttccg accatcaagc attttatccg tactcctgat 1440
gatgcatggt tactcaccac tgcgatcccc gggaaaacag cattccaggt attagaagaa 1500
tatcctgatt caggtgaaaa tattgttgat gcgctggcag tgttcctgcg ccggttgcat 1560
tcgattcctg tttgtaattg tccttttaac agcgatcgcg tatttcgtct cgctcaggcg 1620
caatcacgaa tgaataacgg tttggttgat gcgagtgatt ttgatgacga gcgtaatggc 1680
tggcctgttg aacaagtctg gaaagaaatg cataagcttt tgccattctc accggattca 1740
gtcgtcactc atggtgattt ctcacttgat aaccttattt ttgacgaggg gaaattaata 1800
ggttgtattg atgttggacg agtcggaatc gcagaccgat accaggatct tgccatccta 1860
tggaactgcc tcggtgagtt ttctccttca ttacagaaac ggctttttca aaaatatggt 1920
attgataatc ctgatatgaa taaattgcag tttcatttga tgctcgatga gtttttctaa 1980
acgcgtgacc aagtttactc atatgtactt tagattgatt taaaacttca tttttaattt 2040
aaaaggatct aggtgaagat cctttttgat aatctcatga ccaaaatccc ttaacgtgag 2100
ttttcgttcc actgagcgtc agaccccgta gaaaagatca aaggatcttc ttgagatcct 2160
tttttctgc gcgtaatctg ctgcttgcaa acaaaaaaac caccgctacc agcggtggtt 2220
tgtttgccgg atcaagagct accaactctt tttccgaagg taactggctt cagcagagcg 2280
cagataccaa atactgtcct tctagtgtag ccgtagttag gccaccactt caagaactct 2340
gtagcaccgc ctacatacct cgctctgcta atcctgttac cagtggctgc tgccagtggc 2400
gataagtcgt gtcttaccgg gttggactca agacgatagt taccggataa ggcgcagcgg 2460
tcgggctgaa cggggggttc gtgcacacag cccagcttgg agcgaacgac ctacaccgaa 2520
ctgagatacc tacagcgtga gctatgagaa agcgccacgc ttcccgaagg gagaaaggcg 2580
gacaggtatc cggtaagcgg cagggtcgga acaggagagc gcacgaggga gctcccaggg 2640
ggaaacgcct ggtatcttta tagtcctgtc gggtttcgcc acctctgact tgagcgtcga 2700
tttttgtgat gctcgtcagg ggggcggagc ctatggaaaa acgccagcaa cgcggccttt 2760
ttacggttcc tggccttttg ctggcctttt gctcacatgt tctttcctgc gttatcccct 2820
gattctgtgg ataaccgtat taccgccttt gagtgagctg ataccgctcg ccgcagccga 2880
acgaccgagc gcagcgagtc agtgagcgag gaagcggaag agcgcctgat gcggtatttt 2940

ctccttacgc atctgtgcgg tatttcacac cgcatatggt gcactctcag tacaatctgc 3000
tctgatgccg catagttaag ccagtataca ctccgctatc gctacgtgac tgggtcatgg 3060
ctgcgccccg acacccgcca acacccgctg acgcgccctg acgggcttgt ctgctcccgg 3120
catccgctta cagacaagct gtgaccgtct ccgggagctg catgtgtcag aggttttcac 3180
cgtcatcacc gaaacgcgcg aggcagctgc ggtaaagctc atcagcgtgg tcgtgaagcg 3240
attcacagat gtctgcctgt tcatccgcgt ccagctcgtt gagtttctcc agaagcgtta 3300
atgtctggct tctgataaag cgggccatgt taagggcggt ttttcctgt ttggtcactg 3360
atgcctccgt gtaaggggga tttctgttca tgggggtaat gataccgatg aaacgagaga 3420
ggatgctcac gatacgggtt actgatgatg aacatgcccg gttactggaa cgttgtgagg 3480
gtaaacaact ggcggtatgg atgcggcggg accagagaaa aatcactcag ggtcaatgcc 3540
agcgcttcgt taatacagat gtaggtgttc cacagggtag ccagcagcat cctgcgatgc 3600
agatccggaa cataatggtg cagggcgctg acttccgcgt ttccagactt tacgaaacac 3660
ggaaaccgaa gaccattcat gttgttgctc aggtcgcaga cgttttgcag cagcagtcgc 3720
ttcacgttcg ctcgcgtatc ggtgattcat tctgctaacc agtaaggcaa ccccgccagc 3780
ctagccgggt cctcaacgac aggagcacga tcatgcgcac ccgtggccag gacccaacgc 3840
tgcccgagat gcgccgcgtg cggctgctgg agatggcgga cgcgatggat atgttctgcc 3900
aagggttggt ttgcgcattc acagttctcc gcaagaattg attggctcca attcttggag 3960
tggtgaatcc gttagcgagg tgccgccggc ttccattcag gtcgaggtgg cccggctcca 4020
tgcaccgcga cgcaacgcgg ggaggcagac aaggtatagg gcggcgccta caatccatgc 4080
caacccgttc catgtgctcg ccgaggcggc ataaatcgcc gtgacgatca gcggtccaat 4140
gatcgaagtt aggctggtaa gagccgcgag cgatccttga agctgtccct gatggtcgtc 4200
atctacctgc ctggacagca tggcctgcaa cgcgggcatc ccgatgccgc cggaagcgag 4260
aagaatcata atggggaagg ccatccagcc tcgcgtcgcg aacgccagca agacgtagcc 4320
cagcgcgtcg gccgccatgc cggcgataat ggcctgcttc tcgccgaaac gtttggtggc 4380
gggaccagtg acgaaggctt gagcgagggc gtgcaagatt ccgaataccg caagcgacag 4440
gccgatcatc gtcgcgctcc agcgaaagcg gtcctcgccg aaaatgaccc agagcgctgc 4500
cggcacctgt cctacgagtt gcatgataaa gaagacagtc ataagtgcgg cgacgatagt 4560
catgccccgc gcccaccgga aggagctgac tgggttgaag gctctcaagg gcatcggtcg 4620
acgctctccc ttatgcgact cctgcattag gaagcagccc agtagtaggt tgaggccgtt 4680
gagcaccgcc gccgcaagga atggtgcatg caaggagatg gcgcccaaca gtcccccggc 4740
cacggggcct gccaccatac ccacgccgaa acaagcgctc atgagcccga agtggcgagc 4800

ccgatcttcc ccatcggtga tgtcggcgat ataggcgcca gcaaccgcac ctgtggcgcc 4860

ggtgatgccg gccacgatgc gtccggcgta gaggatccgg gcttatcgac tgcacggtgc 4920

accaatgctt ctggcgtcag gcagccatcg gaagctgtgg tat 4963

<210> 31
<211> 6
<212> DNA
<213> artificial sequence

<220>
<223> Stop Codon

<400> 31
tgaaca 6

<210> 32
<211> 6
<212> DNA
<213> artificial sequence

<220>
<223> Stop Codon

<400> 32
taatga 6

<210> 33
<211> 4525
<212> DNA
<213> artificial sequence

<220>
<223> Plasmid

<400> 33
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgaatt cagacatgca tttcatcctt 420

agttactaag cacgaggaac gactatcacg gcttgaatag gacatttagt cttatcaaac 480

ttagtgaagt caaacggtat ggcaccacca ctggatggat cgcgccaaaa gccaactatc 540

acgccatcgg cgtgataggc cgcaatatcg ctaagagagc aataagcatt tatcgactta 600

agataaatga atcgctcaat gttaccgaaa gcaccaggtt tcttgccctc gcgaatatca 660

tacttctgat cacgcatagc ctgatcagta gcaagataag tcgcatcaag gtcaatataa 720
ccattgccac ggcaaccgcg gaacgtggta taactaagcc gagaatccca atcacgccac 780
tttgtattgc ccaaaagatc tttgagggca acatcaaatt cgcgaggctg gagttcaaca 840
gcattataga taggccacgg tcggttctta gtaggaggct cgtaaacctc ctctagggac 900
caaattgcga agggacaggt atacttacct gtccctggcg gcggatcaat cggtggatcc 960
ggaataaccg gatcgggttt tgaccctgag ccaccaccgg caatgagcag tcattaatac 1020
gctgggttca gctgatcaat agcatcgatc agcagaggac tagcgagcag agcagcaagc 1080
tctgtacgaa caaaagctcg ttcctcatcg gtactatact gcgtgaacga aaaggtcacg 1140
tcagcatatg cctggcgagt aacggatggg tcacaagaac cgtttgcagt gcaagcggtc 1200
gggttctgga tcttaacctg gaccttgtag ttcttacgat tgcgagaagg ctgagatacc 1260
gaaacggtaa cacgcttctc cagcgcagga actgcacccg cttgtgaaag cgaggcaacg 1320
ccgttagtgg gatttacccc acgcggattg aggaccagag tttgttttcc atctttcccg 1380
atgttaccta aagtaacagt ctctaatttt gccatcgttt tttacctcct tctagagtca 1440
ttatggtttt gccatacatc agtatggtgt agcagcactt attataatct ttattgcctc 1500
ttaaaactta atccacatca aaactcaaat acttttaacc ccagcgtcct gtaagctctg 1560
cattaatgaa tcggccaacg cgcggggaga ggcggtttgc gtattgggcg ctcttccgct 1620
tcctcgctca ctgactcgct gcgctcggtc gttcggctgc ggcgagcggt atcagctcac 1680
tcaaaggcgg taatacggtt atccacagaa tcaggggata acgcaggaaa gaacatgtga 1740
gcaaaaggcc agcaaaaggc caggaaccgt aaaaaggccg cgttgctggc gtttttccat 1800
aggctccgcc cccctgacga gcatcacaaa aatcgacgct caagtcagag gtggcgaaac 1860
ccgacaggac tataaagata ccaggcgttt ccccctggaa gctccctcgt gcgctctcct 1920
gttccgaccc tgccgcttac cggatacctg tccgcctttc tcccttcggg aagcgtggcg 1980
ctttctcata gctcacgctg taggtatctc agttcggtgt aggtcgttcg ctccaagctg 2040
ggctgtgtgc acgaaccccc cgttcagccc gaccgctgcg ccttatccgg taactatcgt 2100
cttgagtcca acccggtaag acacgactta tcgccactgg cagcagccac tggtaacagg 2160
attagcagag cgaggtatgt aggcggtgct acagagttct tgaagtggtg gcctaactac 2220
ggctacacta gaagaacagt atttggtatc tgcgctctgc tgaagccagt taccttcgga 2280
aaaagagttg gtagctcttg atccggcaaa caaaccaccg ctggtagcgg tggttttttt 2340
gtttgcaagc agcagattac gcgcagaaaa aaaggatctc aagaagatcc tttgatcttt 2400
tctacggggt ctgacgctca gtggaacgaa aactcacgtt aagggatttt ggtcatgaga 2460
ttatcaaaaa ggatcttcac ctagatcctt ttaaattaaa aatgaagttt taaatcaatc 2520

taaagtatat atgagtaaac ttggtctgac agttaccaat gcttaatcag tgaggcacct 2580

atctcagcga tctgtctatt tcgttcatcc atagttgcct gactccccgt cgtgtagata 2640

actacgatac gggagggctt accatctggc cccagtgctg caatgatacc gcgagaccca 2700

cgctcaccgg ctccagattt atcagcaata aaccagccag ccggaagggc cgagcgcaga 2760

agtggtcctg caactttatc cgcctccatc cagtctatta attgttgccg ggaagctaga 2820

gtaagtagtt cgccagttaa tagtttgcgc aacgttgttg ccattgctac aggcatcgtg 2880

gtgtcacgct cgtcgtttgg tatggcttca ttcagctccg gttcccaacg atcaaggcga 2940

gttacatgat cccccatgtt gtgcaaaaaa gcggttagct ccttcggtcc tccgatcgtt 3000

gtcagaagta agttggccgc agtgttatca ctcatggtta tggcagcact gcataattct 3060

cttactgtca tgccatccgt aagatgcttt tctgtgactg gtgagtgggg gggggggggcg 3120

ctgaggtctg cctcgtgaag aaggtgttgc tgactcatac caggcctgaa tcgccccatc 3180

atccagccag aaagtgaggg agccacggtt gatgagagct ttgttgtagg tggaccagtt 3240

ggtgattttg aacttttgct ttgccacgga acggtctgcg ttgtcgggaa gatgcgtgat 3300

ctgatccttc aactcagcaa aagttcgatt tattcaacaa agccgccgtc ccgtcaagtc 3360

agcgtaatgc tctgccagtg ttacaaccaa ttaaccaatt ctgattagaa aaactcatcg 3420

agcatcaaat gaaactgcaa tttattcata tcaggattat caataccata tttttgaaaa 3480

agccgtttct gtaatgaagg agaaaactca ccgaggcagt tccataggat ggcaagatcc 3540

tggtatcggt ctgcgattcc gactcgtcca acatcaatac aacctattaa tttcccctcg 3600

tcaaaaataa ggttatcaag tgagaaatca ccatgagtga cgactgaatc cggtgagaat 3660

ggcaaaagct tatgcatttc tttccagact tgttcaacag gccagccatt acgctcgtca 3720

tcaaaatcac tcgcatcaac caaaccgtta ttcattcgtg attgcgcctg agcgagacga 3780

aatacgcgat cgctgttaaa aggacaatta caaacaggaa tcgaatgcaa ccggcgcagg 3840

aacactgcca gcgcatcaac aatattttca cctgaatcag gatattcttc taatacctgg 3900

aatgctgttt tcccggggat cgcagtggtg agtaaccatg catcatcagg agtacggata 3960

aaatgcttga tggtcggaag aggcataaat tccgtcagcc agtttagtct gaccatctca 4020

tctgtaacat cattggcaac gctacctttg ccatgtttca gaaacaactc tggcgcatcg 4080

ggcttcccat acaatcgata gattgtcgca cctgattgcc cgacattatc gcgagcccat 4140

ttatacccat ataaatcagc atccatgttg gaatttaatc gcggcctcga gcaagacgtt 4200

tcccgttgaa tatggctcat aacacccctt gtattactgt ttatgtaagc agacagtttt 4260

attgttcatg atgatatatt tttatcttgt gcaatgtaac atcagagatt ttgagacaca 4320

acgtggcttt cccccccccc ccattattga agcatttatc agggttattg tctcatgagc 4380

ggatacatat ttgaatgtat ttagaaaaat aaacaaatag gggttccgcg cacatttccc 4440

cgaaaagtgc cacctgacgt ctaagaaacc attattatca tgacattaac ctataaaaat 4500

aggcgtatca cgaggccctt tcgtc 4525

<210> 34
<211> 56
<212> DNA
<213> artificial sequence

<220>
<223> Primer Sequence

<400> 34
gcgcgcgaat tcaggaggta aaaaacgatg gcaaaattag agactgttac tttagg 56

<210> 35
<211> 33
<212> DNA
<213> artificial sequence

<220>
<223> Primer Sequence

<400> 35
gcatgcaagc ttagacatgc atttcatcct tag 33

<210> 36
<211> 57
<212> DNA
<213> artificial sequence

<220>
<223> Primer Sequence

<400> 36
gcgcgcgaat tctaaggagg aaaaaaaaat ggcaaaatta gagactgtta ctttagg 57

<210> 37
<211> 3914
<212> DNA
<213> artificial sequence

<220>
<223> Cloning Vector

<400> 37
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

```
tacgccagct ggcgaaaggg ggatgtgctg caaggcgatt aagttgggta acgccagggt     360
tttcccagtc acgacgttgt aaaacgacgg ccagtgaatt ccccggatcc gtcgacctgc     420
aggggggggg gggcgctgag gtctgcctcg tgaagaaggt gttgctgact cataccaggc     480
ctgaatcgcc ccatcatcca gccagaaagt gagggagcca cggttgatga gagctttgtt     540
gtaggtggac cagttggtga ttttgaactt ttgctttgcc acggaacggt ctgcgttgtc     600
gggaagatgc gtgatctgat ccttcaactc agcaaaagtt cgatttattc aacaaagccg     660
ccgtcccgtc aagtcagcgt aatgctctgc cagtgttaca accaattaac caattctgat     720
tagaaaaact catcgagcat caaatgaaac tgcaatttat tcatatcagg attatcaata     780
ccatattttt gaaaaagccg tttctgtaat gaaggagaaa actcaccgag gcagttccat     840
aggatggcaa gatcctggta tcggtctgcg attccgactc gtccaacatc aatacaacct     900
attaatttcc cctcgtcaaa aataaggtta tcaagtgaga aatcaccatg agtgacgact     960
gaatccggtg agaatggcaa aagcttatgc atttctttcc agacttgttc aacaggccag    1020
ccattacgct cgtcatcaaa atcactcgca tcaaccaaac cgttattcat tcgtgattgc    1080
gcctgagcga gacgaaatac gcgatcgctg ttaaaaggac aattacaaac aggaatcgaa    1140
tgcaaccggc gcaggaacac tgccagcgca tcaacaatat tttcacctga atcaggatat    1200
tcttctaata cctggaatgc tgttttcccg gggatcgcag tggtgagtaa ccatgcatca    1260
tcaggagtac ggataaaatg cttgatggtc ggaagaggca taaattccgt cagccagttt    1320
agtctgacca tctcatctgt aacatcattg gcaacgctac ctttgccatg tttcagaaac    1380
aactctggcg catcgggctt cccatacaat cgatagattg tcgcacctga ttgcccgaca    1440
ttatcgcgag cccatttata cccatataaa tcagcatcca tgttggaatt taatcgcggc    1500
ctcgagcaag acgtttcccg ttgaatatgg ctcataacac cccttgtatt actgtttatg    1560
taagcagaca gttttattgt tcatgatgat atatttttat cttgtgcaat gtaacatcag    1620
agattttgag acacaacgtg gctttccccc cccccctgc aggtcgacgg atccggggaa    1680
ttcgtaatca tggtcatagc tgtttcctgt gtgaaattgt tatccgctca caattccaca    1740
caacatacga gccggaagca taaagtgtaa agcctggggt gcctaatgag tgagctaact    1800
cacattaatt gcgttgcgct cactgcccgc tttccagtcg ggaaacctgt cgtgccagct    1860
gcattaatga atcggccaac gcgcggggag aggcggtttg cgtattgggc gctcttccgc    1920
ttcctcgctc actgactcgc tgcgctcggt cgttcggctg cggcgagcgg tatcagctca    1980
ctcaaaggcg gtaatacggt tatccacaga atcaggggat aacgcaggaa agaacatgtg    2040
agcaaaaggc cagcaaaagg ccaggaaccg taaaaaggcc gcgttgctgg cgtttttcca    2100
taggctccgc cccccctgacg agcatcacaa aaatcgacgc tcaagtcaga ggtggcgaaa    2160
```

cccgacagga ctataaagat accaggcgtt tccccctgga agctccctcg tgcgctctcc 2220
tgttccgacc ctgccgctta ccggatacct gtccgccttt ctcccttcgg gaagcgtggc 2280
gctttctcaa tgctcacgct gtaggtatct cagttcggtg taggtcgttc gctccaagct 2340
gggctgtgtg cacgaacccc ccgttcagcc cgaccgctgc gccttatccg gtaactatcg 2400
tcttgagtcc aacccggtaa gacacgactt atcgccactg gcagcagcca ctggtaacag 2460
gattagcaga gcgaggtatg taggcggtgc tacagagttc ttgaagtggt ggcctaacta 2520
cggctacact agaaggacag tatttggtat ctgcgctctg ctgaagccag ttaccttcgg 2580
aaaaagagtt ggtagctctt gatccggcaa acaaaccacc gctggtagcg gtggtttttt 2640
tgtttgcaag cagcagatta cgcgcagaaa aaaaggatct caagaagatc ctttgatctt 2700
ttctacgggg tctgacgctc agtggaacga aaactcacgt taagggattt tggtcatgag 2760
attatcaaaa aggatcttca cctagatcct tttaaattaa aaatgaagtt ttaaatcaat 2820
ctaaagtata tatgagtaaa cttggtctga cagttaccaa tgcttaatca gtgaggcacc 2880
tatctcagcg atctgtctat ttcgttcatc catagttgcc tgactccccg tcgtgtagat 2940
aactacgata cgggagggct taccatctgg ccccagtgct gcaatgatac cgcgagaccc 3000
acgctcaccg gctccagatt tatcagcaat aaaccagcca gccggaaggg ccgagcgcag 3060
aagtggtcct gcaactttat ccgcctccat ccagtctatt aattgttgcc gggaagctag 3120
agtaagtagt tcgccagtta atagtttgcg caacgttgtt gccattgcta caggcatcgt 3180
ggtgtcacgc tcgtcgtttg gtatggcttc attcagctcc ggttcccaac gatcaaggcg 3240
agttacatga tcccccatgt tgtgcaaaaa agcggttagc tccttcggtc ctccgatcgt 3300
tgtcagaagt aagttggccg cagtgttatc actcatggtt atggcagcac tgcataattc 3360
tcttactgtc atgccatccg taagatgctt ttctgtgact ggtgagtact caaccaagtc 3420
attctgagaa tagtgtatgc ggcgaccgag ttgctcttgc ccggcgtcaa tacgggataa 3480
taccgcgcca catagcagaa ctttaaaagt gctcatcatt ggaaaacgtt cttcggggcg 3540
aaaactctca aggatcttac cgctgttgag atccagttcg atgtaaccca ctcgtgcacc 3600
caactgatct tcagcatctt ttactttcac cagcgtttct gggtgagcaa aaacaggaag 3660
gcaaaatgcc gcaaaaaagg gaataagggc gacacggaaa tgttgaatac tcatactctt 3720
cctttttcaa tattattgaa gcatttatca gggttattgt ctcatgagcg gatacatatt 3780
tgaatgtatt tagaaaaata aacaaatagg ggttccgcgc acatttcccc gaaaagtgcc 3840
acctgacgtc taagaaacca ttattatcat gacattaacc tataaaaata ggcgtatcac 3900
gaggcccttt cgtc 3914

<210> 38

<211> 57
<212> DNA
<213> artificial sequence

<220>
<223> Primer sequence

<400> 38
gcgcgcgaat tctaaggagg aaaaaaaaat ggcaaataag ccaatgcaac cgatcac 57

<210> 39
<211> 44
<212> DNA
<213> artificial sequence

<220>
<223> Primer Sequence

<400> 39
gcatgcaagc ttcattaagc agtagtatca gacgatacga tagc 44

<210> 40
<211> 4962
<212> DNA
<213> artificial sequence

<220>
<223> Expression Construct

<400> 40
ggctgtgcag gtcgtaaatc actgcataat tcgtgtcgct caaggcgcac tcccgttctg 60

gataatgttt tttgcgccga catcataacg gttctggcaa atattctgaa atgagctgtt 120

gacaattaat catcggctcg tataatgtgt ggaattgtga gcggataaca atttcacaca 180

ggaaacagaa ttcaggaggt aaaaaacgat ggcaaataag ccaatgcaac cgatcacatc 240

tacagcaaat aaaattgtgt ggtcggatcc aactcgttta tcaactacat tttcagcaag 300

tctgttacgc caacgtgtta aagttggtat agccgaactg aataatgttt caggtcaata 360

tgtatctgtt tataagcgtc ctgcacctaa accggaaggt tgtgcagatg cctgtgtcat 420

tatgccgaat gaaaaccaat ccattcgcac agtgatttca gggtcagccg aaaacttggc 480

taccttaaaa gcagaatggg aaactcacaa acgtaacgtt gacacactct tcgcgagcgg 540

caacgccggt ttgggtttcc ttgaccctac tgcggctatc gtatcgtctg atactactgc 600

ttaatgaagc ttggctgttt tggcggatga gagaagattt tcagcctgat acagattaaa 660

tcagaacgca gaagcggtct gataaaacag aatttgcctg gcggcagtag cgcggtggtc 720

ccacctgacc ccatgccgaa ctcagaagtg aaacgccgta gcgccgatgg tagtgtgggg 780

tctccccatg cgagagtagg gaactgccag gcatcaaata aaacgaaagg ctcagtcgaa 840

agactgggcc tttcgtttta tctgttgttt gtcggtgaac gctctcctga gtaggacaaa 900

tccgccggga gcggatttga acgttgcgaa gcaacggccc ggagggtggc gggcaggacg 960
cccgccataa actgccaggc atcaaattaa gcagaaggcc atcctgacgg atggcctttt 1020
tgcgtttcta caaactcttt tgtttatttt tctagagcca cgttgtgtct caaaatctct 1080
gatgttacat tgcacaagat aaaaatatat catcatgaac aataaaactg tctgcttaca 1140
taaacagtaa tacaaggagt gttatgagcc atattcaacg ggaaacgtct tgctcgaggc 1200
cgcgattaaa ttccaacatg gatgctgatt tatatgggta taaatgggct cgcgataatg 1260
tcgggcaatc aggtgcgaca atctatcgat tgtatgggaa gcccgatgcg ccagagttgt 1320
ttctgaaaca tggcaaaggt agcgttgcca atgatgttac agatgagatg gtcagactaa 1380
actggctgac ggaatttatg cctcttccga ccatcaagca ttttatccgt actcctgatg 1440
atgcatggtt actcaccact gcgatccccg ggaaaacagc attccaggta ttagaagaat 1500
atcctgattc aggtgaaaat attgttgatg cgctggcagt gttcctgcgc cggttgcatt 1560
cgattcctgt ttgtaattgt ccttttaaca gcgatcgcgt atttcgtctc gctcaggcgc 1620
aatcacgaat gaataacggt ttggttgatg cgagtgattt tgatgacgag cgtaatggct 1680
ggcctgttga acaagtctgg aaagaaatgc ataagctttt gccattctca ccggattcag 1740
tcgtcactca tggtgatttc tcacttgata accttatttt tgacgagggg aaattaatag 1800
gttgtattga tgttggacga gtcggaatcg cagaccgata ccaggatctt gccatcctat 1860
ggaactgcct cggtgagttt tctccttcat tacagaaacg gctttttcaa aaatatggta 1920
ttgataatcc tgatatgaat aaattgcagt ttcatttgat gctcgatgag tttttctaaa 1980
cgcgtgacca agtttactca tatgtacttt agattgattt aaaacttcat ttttaattta 2040
aaaggatcta ggtgaagatc ctttttgata atctcatgac caaaatccct taacgtgagt 2100
tttcgttcca ctgagcgtca gaccccgtag aaaagatcaa aggatcttct tgagatcctt 2160
tttttctgcg cgtaatctgc tgcttgcaaa caaaaaaacc accgctacca gcggtggttt 2220
gtttgccgga tcaagagcta ccaactcttt ttccgaaggt aactggcttc agcagagcgc 2280
agataccaaa tactgtcctt ctagtgtagc cgtagttagg ccaccacttc aagaactctg 2340
tagcaccgcc tacatacctc gctctgctaa tcctgttacc agtggctgct gccagtggcg 2400
ataagtcgtg tcttaccggg ttggactcaa gacgatagtt accggataag gcgcagcggt 2460
cgggctgaac ggggggttcg tgcacacagc ccagcttgga gcgaacgacc tacaccgaac 2520
tgagatacct acagcgtgag ctatgagaaa gcgccacgct tcccgaaggg agaaaggcgg 2580
acaggtatcc ggtaagcggc agggtcggaa caggagagcg cacgagggag ctcccagggg 2640
gaaacgcctg gtatctttat agtcctgtcg ggtttcgcca cctctgactt gagcgtcgat 2700
ttttgtgatg ctcgtcaggg gggcggagcc tatggaaaaa cgccagcaac gcggcctttt 2760

tacggttcct ggccttttgc tggccttttg ctcacatgtt ctttcctgcg ttatcccctg 2820
attctgtgga taaccgtatt accgcctttg agtgagctga taccgctcgc cgcagccgaa 2880
cgaccgagcg cagcgagtca gtgagcgagg aagcggaaga gcgcctgatg cggtattttc 2940
tccttacgca tctgtgcggt atttcacacc gcatatggtg cactctcagt acaatctgct 3000
ctgatgccgc atagttaagc cagtatacac tccgctatcg ctacgtgact gggtcatggc 3060
tgcgccccga cacccgccaa cacccgctga cgcgccctga cgggcttgtc tgctcccggc 3120
atccgcttac agacaagctg tgaccgtctc cgggagctgc atgtgtcaga ggttttcacc 3180
gtcatcaccg aaacgcgcga ggcagctgcg gtaaagctca tcagcgtggt cgtgaagcga 3240
ttcacagatg tctgcctgtt catccgcgtc cagctcgttg agtttctcca gaagcgttaa 3300
tgtctggctt ctgataaagc gggccatgtt aagggcggtt ttttcctgtt tggtcactga 3360
tgcctccgtg taagggggat ttctgttcat gggggtaatg ataccgatga aacgagagag 3420
gatgctcacg atacgggtta ctgatgatga acatgcccgg ttactggaac gttgtgaggg 3480
taaacaactg gcggtatgga tgcggcggga ccagagaaaa atcactcagg gtcaatgcca 3540
gcgcttcgtt aatacagatg taggtgttcc acagggtagc cagcagcatc ctgcgatgca 3600
gatccggaac ataatggtgc agggcgctga cttccgcgtt tccagacttt acgaaacacg 3660
gaaaccgaag accattcatg ttgttgctca ggtcgcagac gttttgcagc agcagtcgct 3720
tcacgttcgc tcgcgtatcg gtgattcatt ctgctaacca gtaaggcaac cccgccagcc 3780
tagccgggtc ctcaacgaca ggagcacgat catgcgcacc cgtggccagg acccaacgct 3840
gcccgagatg cgccgcgtgc ggctgctgga gatggcggac gcgatggata tgttctgcca 3900
agggttggtt tgcgcattca cagttctccg caagaattga ttggctccaa ttcttggagt 3960
ggtgaatccg ttagcgaggt gccgccggct tccattcagg tcgaggtggc ccggctccat 4020
gcaccgcgac gcaacgcggg gaggcagaca aggtataggg cggcgcctac aatccatgcc 4080
aacccgttcc atgtgctcgc cgaggcggca taaatcgccg tgacgatcag cggtccaatg 4140
atcgaagtta ggctggtaag agccgcgagc gatccttgaa gctgtccctg atggtcgtca 4200
tctacctgcc tggacagcat ggcctgcaac gcgggcatcc cgatgccgcc ggaagcgaga 4260
agaatcataa tggggaaggc catccagcct cgcgtcgcga acgccagcaa gacgtagccc 4320
agcgcgtcgg ccgccatgcc ggcgataatg gcctgcttct cgccgaaacg tttggtggcg 4380
ggaccagtga cgaaggcttg agcgagggcg tgcaagattc cgaataccgc aagcgacagg 4440
ccgatcatcg tcgcgctcca gcgaaagcgg tcctcgccga aaatgaccca gagcgctgcc 4500
ggcacctgtc ctacgagttg catgataaag aagacagtca taagtgcggc gacgatagtc 4560
atgccccgcg cccaccggaa ggagctgact gggttgaagg ctctcaaggg catcggtcga 4620

```
cgctctccct tatgcgactc ctgcattagg aagcagccca gtagtaggtt gaggccgttg   4680
agcaccgccg ccgcaaggaa tggtgcatgc aaggagatgg cgcccaacag tcccccggcc   4740
acggggcctg ccaccatacc cacgccgaaa caagcgctca tgagcccgaa gtggcgagcc   4800
cgatcttccc catcggtgat gtcggcgata taggcgccag caaccgcacc tgtggcgccg   4860
gtgatgccgg ccacgatgcg tccggcgtag aggatccggg cttatcgact gcacggtgca   4920
ccaatgcttc tggcgtcagg cagccatcgg aagctgtggt at                      4962
```